# EUROPEAN PATENT SPECIFICATION

(11) **EP 2 490 761 B1**
(45) Date of publication and mention of the grant of the patent: **19.08.2015**
(21) Application number: 10774043.3
(22) Date of filing: 14.10.2010
(51) Int. Cl.: A61N 1/372

(54) **ASSIGNMENT AND MANIPULATION OF IMPLANTABLE LEADS IN DIFFERENT ANATOMICAL REGIONS WITH IMAGE BACKGROUND**
BESTIMMUNG UND BEDIENUNG VON IMPLANTIERBAREN ADERN IN VERSCHIEDENEN ANATOMISCHEN REGIONEN MIT HINTERGRUNDBILD
ATTRIBUTION ET MANIPULATION DES FILS IMPLANTABLES DANS DIFFÉRENTES RÉGIONS ANATOMIQUES AVEC IMAGE DE FOND

(30) Priority: 21.10.2009 US 253759 P; 21.10.2009 US 253766 P; 21.10.2009 US 253756 P; 12.11.2009 US 260712 P; 12.11.2009 US 260644 P; 12.11.2009 US 260707 P; 30.04.2010 US 771763
(43) Date of publication of application: 29.08.2012
(73) Proprietor: Medtronic, Inc., Minneapolis, MN 55432 (US)
(72) Inventor: DAVIS, Jon, P., St. Michael, Minnesota 55376 (US); GOETZ, Steven, M., North Oaks, Minnesota 55127 (US); SINGAL, Ashish, Blaine, Minnesota 55449 (US); DAVENPORT, Lynn, A., New Brighton, MN 55112 (US); BOURGET, Dennis, J., St. Michael, Minnesota 55376 (US); SAHASRABUDHE, Rajeev, Maple Grove, Minnesota 55311 (US); HUHTA, Brent, A., Big Lake, Minnesota 55309 (US); KANDIKONDA, Shanthi, Woodbury, Minnesota 55129 (US); RAHN, Jason, D., Andover, Minnesota 55304 (US)
(74) Representative: Hughes, Andrea Michelle
(86) International application number: PCT/US2010/052573
(87) International publication number: WO 2011/049802

(56) References cited:
- US-A1- 2004 215 239
- US-A1- 2005 131 474
- US-A1- 2007 255 339
- US-A1- 2009 012 416
- US-A1- 2009 083 070

## Description

### TECHNICAL FIELD

The disclosure relates to medical devices and, more particularly, to medical devices that deliver electrical stimulation therapy.

### BACKGROUND

Medical devices may be used to treat a variety of medical conditions. Medical electrical stimulation devices, for example, may deliver electrical stimulation therapy to a patient via implanted electrodes. Electrical stimulation therapy may include stimulation of nerve, muscle, or brain tissue, or other tissue within a patient. An electrical stimulation device may be fully implanted within the patient. For example, an electrical stimulation device may include an implantable electrical stimulation generator and one or more implantable leads carrying electrodes. Alternatively, the electrical stimulation device may comprise a leadless stimulator. In some cases, implantable electrodes may be coupled to an external electrical stimulation generator via one or more percutaneous leads or fully implanted leads.

Medical electrical stimulators may be used to deliver electrical stimulation therapy to patients to relieve a variety of symptoms or conditions such as chronic pain, tremor, Parkinson's disease, depression, epilepsy, urinary or fecal incontinence, pelvic pain, sexual dysfunction, obesity, or gastroparesis. An electrical stimulator may be configured to deliver electrical stimulation therapy via leads that include electrodes implantable proximate to the spinal cord, pelvic nerves, gastrointestinal organs, peripheral nerves, or within the brain of a patient. Stimulation proximate the spinal cord and within the brain are often referred to as spinal cord stimulation (SCS) and deep brain stimulation (DBS), respectively.

A clinician selects values for a number of programmable stimulation parameters in order to define the electrical stimulation therapy to be delivered to a patient. For example, the clinician may select a current or voltage amplitude of the stimulation, and various characteristics of the stimulation waveform. In addition, the clinician may specify an electrode configuration used to deliver stimulation, including selected electrode combinations and electrode polarities. If the stimulation is delivered in the form of pulses, for example, the clinician may specify a pulse width and pulse rate. A set of parameter values may be referred to as a stimulation program. A program group may include multiple programs. Multiple programs in a program group may be delivered on a simultaneous, time-interleaved, or overlapping basis.

US 2009/0012416, US 2004/0215239, US 2005/0131474, US 2009/0083070 and US 2007/0288070 all teach interfaces for patient and/or physician monitoring of implantable medical devices relative to the patient.

### SUMMARY

Generally, this disclosure describes techniques for creating, assigning, and manipulating implanted leads in different anatomical regions utilizing a graphical view of the leads and an image of the regions to which the leads are to deliver electrical stimulation therapy.

In one example, the disclosure is directed to a programmer for an implantable medical device comprising a processor that associates one or more implantable elements of an implantable medical device with one or more anatomical implant regions of a patient, and a user interface that receives user input selecting one of the anatomical implant regions, and receives user input specifying one or more therapy parameters for the one or more implantable elements associated with the selected anatomical region.

In another example, the disclosure is directed to a method comprising associating one or more implantable elements of an implantable medical device with one or more anatomical implant regions of a patient, and receiving user input selecting one of the anatomical implant regions, and receiving user input specifying one or more therapy parameters for the one or more implantable elements associated with the selected anatomical region.

In another example, the disclosure is directed to a device comprising means for associating one or more implantable elements of an implantable medical device with one or more anatomical implant regions of a patient, and means for receiving user input selecting one of the anatomical implant regions, and receiving user input specifying one or more therapy parameters for the one or more implantable elements associated with the selected anatomical region.

In another example, the disclosure is directed to a computer-readable medium comprising instructions that, upon execution, cause a processor to associate one or more implantable elements of an implantable medical device with one or more anatomical implant regions of a patient, and receive user input selecting one of the anatomical implant regions, and receiving user input specifying one or more therapy parameters for the one or more implantable elements associated with the selected anatomical region.

The details of one or more aspects of the disclosure are set forth in the accompanying drawings and the description below. Other features, objects, and advantages will be apparent from the description and drawings, and from the claims.

### BRIEF DESCRIPTION OF DRAWINGS

FIG. 1 is a conceptual diagram illustrating an example therapy system that includes an implantable stimulator coupled to a stimulation lead.
FIG. 2 is a conceptual diagram illustrating another example therapy system that includes an implantable stimulator coupled to a stimulation lead.
FIG. 3 is a block diagram illustrating various example components of an implantable electrical stimulator.
FIG. 4 is a block diagram illustrating various example components of an external programmer.
FIG. 5 is a block diagram illustrating various components of an example electrical stimulation generator for use in the implantable electrical stimulator of FIG 3.
FIGS. 6A-6L illustrate an exemplary sequence of programmer screens, in accordance with this disclosure.
FIG. 7 is a flow diagram illustrating example operation of a programmer to create, assign, and manipulate leads in different regions, in accordance with this disclosure.

### DETAILED DESCRIPTION

This disclosure describes techniques for creating, assigning, and manipulating implanted leads in different anatomical regions utilizing a graphical view of the leads and an image of the regions to which the leads are to deliver electrical stimulation therapy. The examples of this disclosure may provide the user with the ability to accurately define a lead image relative to an anatomical target, which may be helpful in accurately programming stimulation fields. The techniques of this disclosure describe how to organize, assign, and manipulate leads based on an optional anatomical image background.

In one example, the user may utilize a user interface to assign implanted leads to one or more regions in a patient, where the regions represent the physical implant location of the leads in the patient body. The user interface may allow the user to view all the leads, or to view the leads only assigned to a selected region. The graphical representation of the leads, which is presented to the user on the user interface, may be used to represent the leads relative to the physical implantation location. In another example, the user may import an anatomical image, e.g., fluoroscopy image, X-ray image, MRI image, graphical representation of the anatomical region, or the like, which may be then presented in the background as a reference for one or more of the anatomical regions programmed by the user. The user may also utilize the user interface to manipulate the graphical representation of the leads to match the leads' actual implanted orientation at the implant location for one or more regions. In some examples, the actual leads may be visible on the imported image in the background, and utilized as a reference to adjust the graphical representation of the leads to match the actual leads' orientation and position. In one example, the leads in each region may be programmed and manipulated individually based on the region, and independently from the other regions of the patient.

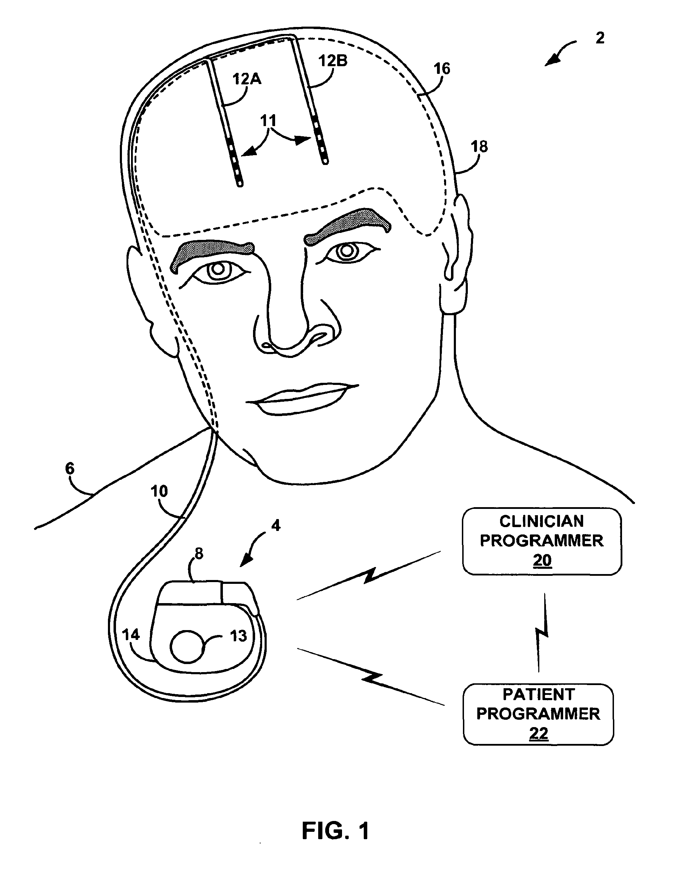

FIG. 1 is a conceptual diagram illustrating an example system 2 including an implantable electrical stimulator 4 that may be used to deliver stimulation therapy to patient 6. Patient 6 ordinarily, but not necessarily, will be a human. Generally, therapy system 2 includes implantable electrical stimulator 4 that delivers electrical stimulation to patient 6 via one or more implantable electrodes 11. The implantable electrodes 11 may be deployed on one or more implantable medical leads, such as implantable medical lead 10, and in some cases on a can electrode. The electrical stimulation may be in the form of controlled current or voltage pulses or substantially continuous waveforms.

Various parameters of the pulses or waveforms may be defined by one or more stimulation program. The pulses or waveforms may be delivered substantially continuously or in bursts, segments, or patterns, and may be delivered alone or in combination with pulses or waveforms defined by one or more other stimulation programs. Although FIG. 1 shows a fully implantable stimulator 4, techniques described in this disclosure may be applied to external stimulators having electrodes deployed via percutaneously implantable leads with a patch electrode or other indifferent electrode attached externally to serve as the can or case. One or more of the electrodes may be located on a housing 14, i.e., "can" or "case," of the implantable stimulator 4. In addition, in some cases, implantable electrodes may be deployed on a leadless stimulator.

In the example illustrated in FIG. 1, implantable stimulator 4 is implanted within a subcutaneous pocket in a clavicle region of patient 6. Stimulator 4 generates programmable electrical stimulation, e.g., a current waveform or current pulses, and delivers the stimulation via an implantable medical lead 10 carrying an array of implantable stimulation electrodes 11. In some cases, multiple implantable leads may be provided. In the example of FIG. 1, a distal end of lead 10 is bifurcated and includes two lead segments 12A and 12B (collectively "lead segments 12"). Lead segments 12A and 12B each include a set of electrodes forming part of the array of electrodes 11. In various examples, lead segments 12A and 12B may each carry four, eight, twelve, sixteen, or more electrodes. In FIG. 1, each lead segment 12A, 12B carries four electrodes, configured as ring electrodes at different axial positions near the distal ends of the lead segments. Throughout the remainder of this disclosure, for purposes of simplicity, the disclosure may generally refer to electrodes carried on "leads" rather than "lead segments."

FIG. 1 further depicts a housing, or can, electrode 13. Housing electrode 13 may be formed integrally with an outer surface of hermetically-sealed housing 14 of implantable stimulator 4, also referred to in this disclosure as implantable medical device (IMD) 4, or otherwise coupled to housing 14. In one example, housing electrode 13 may be described as an active, non-detachable electrode on the surface of the IMD. In some examples, housing electrode 13 is defined by an uninsulated portion of an outward facing portion of housing 14 of IMD 4. Other divisions between insulated and uninsulated portions of housing 14 may be employed to define two or more housing electrodes, which may be referred to as case or can electrodes. In some examples, housing electrode 13 comprises substantially all of housing 14, one side of housing 14, a portion of the housing 14, or multiple portions of housing 14. In other examples, electrode 13 may be formed by an electrode on a dedicated short lead extending from housing 14. As a further alternative, housing electrode 13 may be provided on a proximal portion of one of the leads carrying electrodes 11. The proximal portion may be closely adjacent to housing 14, e.g., at or near a point at which lead 10 is coupled to the housing, such as adjacent to a lead connection header 8 of the housing. In another example, a patch electrode or other indifferent electrode may be attached externally to serve as the can or case.

In some examples, lead 10 may also carry one or more sense electrodes to permit implantable stimulator 4 to sense electrical signals from patient 6. Some of the stimulation electrodes may be coupled to function as stimulation electrodes and sense electrodes on a selective basis. In other examples, implantable stimulator 4 may be coupled to one or more leads which may or may not be bifurcated. In such examples, the leads may be coupled to implantable stimulator 4 via a common lead extension or via separate lead extensions.

A proximal end of lead 10 may be both electrically and mechanically coupled to header 8 on implantable stimulator 4 either directly or indirectly via a lead extension. Conductors in the lead body may electrically connect stimulation electrodes located on lead segments 12 to implantable stimulator 4. Lead 10 traverses from the implant site of implantable stimulator 4 along the neck of patient 6 to cranium 18 of patient 6 to access brain 16. Lead segments 12A and 12B are implanted within the right and left hemispheres, respectively, in order to deliver electrical stimulation to one more regions of brain 16, which may be selected based on the patient condition or disorder.

Implantable stimulator 4 may deliver, for example, deep brain stimulation (DBS) or cortical stimulation (CS) therapy to patient 6 via the electrodes carried by, i.e., located on, lead segments 12 to treat any of a variety of neurological disorders or diseases. Example neurological disorders may include depression, dementia, obsessive-compulsive disorder and movement disorders, such as Parkinson's disease, spasticity, epilepsy, and dystonia. DBS also may be useful for treating other patient conditions, such as migraines and obesity. However, the disclosure is not limited to the configuration of lead 10 shown in FIG 1, or to the delivery of DBS or CS therapy.

Lead segments 12A, 12B may be implanted within a desired location of brain 16 through respective holes in cranium 18. Lead segments 12A, 12B may be placed at any location within brain 16 such that the electrodes located on lead segments 12A, 12B are capable of providing electrical stimulation to targeted tissue during treatment. Example locations for lead segments 12A, 12B within brain 26 may include the pedunculopontine nucleus (PPN), thalamus, basal ganglia structures (e.g., globus pallidus, substantia nigra, subthalmic nucleus), zona inserta, fiber tracts, lenticular fasciculus (and branches thereof), ansa lenticularis, and/or the Field of Forel (thalamic fasciculus). In the case of migraines, lead segments 12 may be implanted to provide stimulation to the visual cortex of brain 16 in order to reduce or eliminate migraine headaches afflicting patient 6. However, the target therapy delivery site may depend upon the patient condition or disorder being treated.

The electrodes of lead segments 12A, 12B are shown as ring electrodes. Ring electrodes are commonly used in DBS applications because they are simple to program and are capable of delivering an electrical field to any tissue adjacent to lead segments 12A, 12B. In other implementations, the electrodes of lead segments 12A, 12B may have different configurations. For example, the electrodes of lead segments 12A, 12B may have a complex electrode array geometry that is capable of producing shaped electrical fields. The complex electrode array geometry may include multiple electrodes (e.g., partial ring or segmented electrodes) around the perimeter of each lead segments 12A, 12B, rather than one ring electrode. In this manner, electrical stimulation may be directed in a specific direction from lead segments 12 to enhance therapy efficacy and reduce possible adverse side effects from stimulating a large volume of tissue. In alternative examples, lead segments 12 may have shapes other than elongated cylinders as shown in FIG. 1. For example, lead segments 12 may be paddle leads, spherical leads, bendable leads, or any other type of shape effective in treating patient 6.

Therapy system 2 also may include a clinician programmer 20 and/or a patient programmer 22. Clinician programmer 20 may be a handheld computing device that permits a clinician to program stimulation therapy for patient 6 via a user interface, e.g., using input keys and a display. For example, using clinician programmer 20, the clinician may specify stimulation parameters, i.e., create programs, for use in delivery of stimulation therapy. Clinician programmer 20 may support telemetry (e.g., radio frequency (RF) telemetry) with implantable stimulator 4 to download programs and, optionally, upload operational or physiological data stored by implantable stimulator 4. In this manner, the clinician may periodically interrogate implantable stimulator 4 to evaluate efficacy and, if necessary, modify the programs or create new programs. In some examples, clinician programmer 20 transmits programs to patient programmer 22 in addition to or instead of implantable stimulator 4. In some examples, patient programmer 22 may serve as the clinician programmer.

Like clinician programmer 20, patient programmer 22 may be a handheld computing device. Patient programmer 22 may also include a display and input keys to allow patient 6 to interact with patient programmer 22 and implantable stimulator 4. In this manner, patient programmer 22 provides patient 6 with a user interface for control of the stimulation therapy delivered by implantable stimulator 4. For example, patient 6 may use patient programmer 22 to start, stop or adjust electrical stimulation therapy. In particular, patient programmer 22 may permit patient 6 to adjust stimulation parameters of a program such as duration, current or voltage amplitude, pulse width and pulse rate. Patient 6 may also select a program, e.g., from among a plurality of stored programs, as the present program to control delivery of stimulation by implantable stimulator 4.

In accordance with various example techniques described in this disclosure, a programmer such as, for example, clinician programmer 20 and/or patient programmer 22 may be used to program leads or lead groups by assigning them to anatomical regions in which they apply the stimulation therapy. The leads or lead groups may be programmed in a region-based manner, where each defined region and its corresponding leads may be programmed independently of any other regions. In one example, a programmer may be used to graphically program the leads or lead groups by defining desired stimulation field(s) within zones on or adjacent to one or more leads, and generating the stimulation that would achieve the stimulation field. In particular, a programmer may be used for translating one or more user input stimulation zones into a set of electrodes for delivering electrical stimulation therapy to a patient, determining the variable electrical stimulation contributions of each electrode to the zone, and determining amplitudes of electrical stimulation when using zone-based programming. A programmer may also be used for graphically representing the stimulation zone and receiving input from a user that manipulates the shape and position of the zone. In another example, a programmer may be used to program the leads or lead groups by defining the stimulation parameters associated with each lead or group of leads, by selecting electrodes and the corresponding parameters, e.g., amplitude, pulse rate, pulse width, etc. In one example, the programmer may be used to program the case electrodes by defining stimulation parameters associated with the case electrodes.

In some examples, implantable stimulator 4 delivers stimulation according to a group of programs at a given time. Each program of such a program group may include respective values for each of a plurality of therapy parameters, such as respective values for each of current or voltage amplitude, pulse width, pulse shape, pulse rate and electrode configuration (e.g., electrode combination and polarity). Implantable stimulator 4 may interleave pulses or other signals according to the different programs of a program group, e.g., cycle through the programs, to simultaneously treat different symptoms or different body regions, or provide a combined therapeutic effect. In such examples, clinician programmer 20 may be used to create programs, and assemble the programs into program groups. Patient programmer 22 may be used to adjust stimulation parameters of one or more programs of a program group, and select a program group, e.g., from among a plurality of stored program groups, as the current program group to control delivery of stimulation by implantable stimulator 4. In another example, the groups of programs may be organized in slots according to the target area of pain, where each slot may be made up of alternative programs with different stimulation parameters targeting the same area of pain, and a patient may change the effective program within a slot, without affecting the selected programs in other slots.

Implantable stimulator 4, clinician programmer 20, and patient programmer 22 may communicate via cables or a wireless communication, as shown in FIG. 1. Clinician programmer 20 and patient programmer 22 may, for example, communicate via wireless communication with implantable stimulator 4 using RF telemetry techniques known in the art or other standard communication protocols such as, for example, Bluetooth^{®}. Clinician programmer 20 and patient programmer 22 also may communicate with each other using any of a variety of wireless communication techniques, such as RF communication according to the 802.11 or Bluetooth specification sets, infrared communication, e.g., according to the IrDA standard, or other standard or proprietary telemetry protocols. Each of clinician programmer 20 and patient programmer 22 may include a transceiver to permit bi-directional communication with implantable stimulator 4.

Generally, system 2 delivers stimulation therapy to patient 6 in the form of controlled current or voltage waveforms or constant current or voltage pulses. The shapes of the pulses may vary according to different design objectives. In the case of current-based stimulation, implantable stimulator 4 regulates current that is sourced or sunk by one or more electrodes, referred to as regulated electrodes. In some examples, one of the electrodes may be unregulated. In such configurations, either the housing electrode or a lead electrode may be the unregulated electrode. Alternatively, all active electrodes may be regulated, i.e., coupled to a current regulator such as a regulated current source or sink.

A source current may refer to a current that flows out of an electrode, e.g., from a regulated current source via a regulated current path to surrounding tissue, or from a reference voltage via an unregulated current path. A sink current may refer to a current that flows into an electrode, e.g. from surrounding tissue and is sunk by a regulated current sink via a regulated current path or by a reference voltage via an unregulated current path. Regulated source currents may sum to produce a greater overall source current. Regulated sink currents may sum to produce a greater overall sink current. Regulated source and regulated sink currents may partially or entirely cancel one another, producing a net difference in the form of a net source current or sink current in the case or partial cancellation. An unregulated current path can source or sink current approximately equal to this net difference.

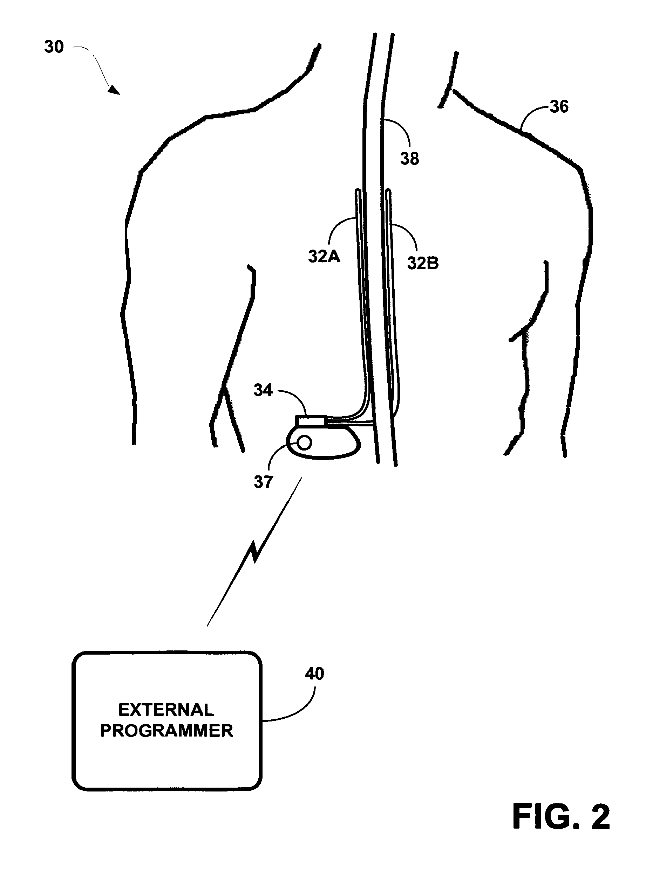

FIG 2 is a conceptual diagram illustrating system 30 that delivers stimulation therapy to spinal cord 38 of patient 36. Other electrical stimulation systems may be configured to deliver electrical stimulation to gastrointestinal organs, pelvic nerves or muscle, peripheral nerves, or other stimulation sites. In the example of FIG. 2, system 30 delivers stimulation therapy from implantable stimulator 34 to spinal cord 38 via one or more electrodes (not shown) carried by, i.e., located on, implantable medical leads 32A and 32B (collectively "leads 32") as well as the housing of implantable stimulator 34, e.g., housing electrode 37. System 30 and, more particularly, implantable stimulator 34 may operate in a manner similar to implantable stimulator 4 (FIG. 1). That is, in a current-based example, implantable stimulator 34 delivers controlled current stimulation pulses or waveforms to patient 36 via one or more regulated, stimulation electrodes. Alternatively, implantable stimulator 34 may be configured to deliver constant voltage pulses. As mentioned above, in some examples, one of the electrodes may be unregulated.

In the example of FIG. 2, the distal ends of leads 32 carry electrodes that are placed adjacent to the target tissue of spinal cord 38. The proximal ends of leads 32 may be both electrically and mechanically coupled to implantable stimulator 34 either directly or indirectly via a lead extension and header. Alternatively, in some examples, leads 32 may be implanted and coupled to an external stimulator, e.g., through a percutaneous port. In additional example implementations, stimulator 34 may be a leadless stimulator with one or more arrays of electrodes arranged on a housing of the stimulator rather than leads that extend from the housing. Application of certain techniques will be described in this disclosure with respect to implantable stimulator 34 and implantable leads 32 having ring electrodes for purposes of illustration. However, other types of electrodes may be used.

Stimulator 34 may be implanted in patient 36 at a location minimally noticeable to the patient. For SCS, stimulator 34 may be located in the lower abdomen, lower back, or other location to secure the stimulator. Leads 32 are tunneled from stimulator 34 through tissue to reach the target tissue adjacent to spinal cord 38 for stimulation delivery. At the distal ends of leads 32 are one or more electrodes (not shown) that transfer the stimulation pulses from the lead to the tissue substantially simultaneously with stimulation pulses. Some of the electrodes may be electrode pads on a paddle lead, circular (i.e., ring) electrodes surrounding the body of leads 32, conformable electrodes, cuff electrodes, segmented electrodes, or any other type of electrodes capable of forming unipolar, bipolar or multi-polar electrode configurations.

The stimulation pulses may be delivered using various electrode arrangements such as unipolar arrangements, bipolar arrangements or multipolar arrangements. A unipolar stimulation arrangement generally refers to the use of an anode on the housing that sources current and one or more cathodes on one or more leads that sink current. A bipolar stimulation arrangement generally refers to the use of an anode on a lead that sources current and a cathode on the same lead and/or another lead that sink current. A multipolar stimulation arrangement generally refers to the use of more than one anode on a lead that each source current and one or more cathodes on the same lead or another lead that sink current, or the use of one anode on a lead that sources current and multiple cathodes on the same lead or another lead that sink current. A hybrid stimulation arrangement that combines both unipolar and bipolar electrode relationships may be referred to as an omnipolar arrangement. In an omnipolar arrangement, an anode on the housing may be used to deliver stimulation pulses substantially simultaneously with at least one anode on a lead and at least one cathode on a lead. In this case, for an omnipolar arrangement, at least one anode on a lead and at least one anode on the housing can be used simultaneously in combination with at least one cathode on a lead. In other omnipolar arrangements, a cathode on the housing may be used to deliver stimulation pulses substantially simultaneously with at least one cathode on a lead and at least one anode on a lead. In this alternative case, for an omnipolar arrangement, at least one cathode on a lead and at least one cathode on the housing can be used simultaneously in combination with at least one anode on a lead. Any of the above electrode arrangements, or other electrode arrangements, may be used to deliver electrical stimulation in accordance with techniques described in this disclosure.

Implantable stimulator 34 delivers stimulation to spinal cord 38 to reduce the amount of pain perceived by patient 36. As mentioned above, however, stimulators may be used with a variety of different therapies, such as peripheral nerve stimulation (PNS), peripheral nerve field stimulation (PNFS), deep brain stimulation (DBS), cortical stimulation (CS), pelvic floor stimulation, peripheral nerve stimulation, gastric stimulation, and the like. The stimulation delivered by implantable stimulator 34 may take the form of stimulation pulses or continuous stimulation waveforms, and may be characterized by controlled current or voltage levels, as well as programmed pulse widths and pulse rates in the case of stimulation current pulses. Stimulation may be delivered via selected combinations of electrodes located on one or both of leads 32 and on the housing. Stimulation of spinal cord 38 may, for example, prevent pain signals from traveling through the spinal cord and to the brain of the patient. Patient 34 perceives the interruption of pain signals as a reduction in pain and, therefore, efficacious therapy.

With reference to FIG. 2, a user, such as a clinician or patient 36, may interact with a user interface of external programmer 40 to program stimulator 34. Programming of stimulator 34 may refer generally to the generation and transfer of commands, programs, or other information to control the operation of the stimulator. For example, programmer 40 may transmit programs, parameter adjustments, program selections, group selections, or other information to control the operation of stimulator 34, e.g., by wireless telemetry.

In some cases, external programmer 40 may be characterized as a physician or clinician programmer, such as clinician programmer 20 (FIG 1), if it is primarily intended for use by a physician or clinician. In other cases, external programmer 40 may be characterized as a patient programmer, such as patient programmer 22 (FIG 1), if it is primarily intended for use by a patient. In general, a physician or clinician programmer may support selection and generation of parameters and/or programs by a clinician for use by stimulator 34, whereas a patient programmer may support adjustment and selection of such parameters and/or programs by a patient during ordinary use.

Whether programmer 40 is configured for clinician or patient use, programmer 40 may communicate to implantable stimulator 34 or any other computing device via wireless communication. Programmer 40, for example, may communicate via wireless communication with implantable stimulator 34 using radio frequency (RF) telemetry techniques known in the art or other communication standards such as, for example, Bluetooth^{®}. Programmer 40 may also communicate with another programmer or computing device via a wired or wireless connection using any of a variety of local wireless communication techniques, such as RF communication according to the 802.11 or Bluetooth^{®} specification sets, infrared communication according to the IRDA specification set, or other standard or proprietary telemetry protocols. Programmer 40 may also communicate with another programming or computing device via exchange of removable media, such as magnetic or optical disks, or memory cards or sticks. Further, programmer 40 may communicate with implantable stimulator 34 and other programming devices via remote telemetry techniques known in the art, communicating via a local area network (LAN), wide area network (WAN), public switched telephone network (PSTN), or cellular telephone network, for example.

In one example, programming of stimulator 34 of FIG 2 may also include graphically defining a desired stimulation field(s) within zones on or adjacent to one or more leads or electrodes, and generating, via a programmer, the current stimulation required to create the stimulation field, e.g., as described with reference to FIG. 1. Programming of stimulator 34 may also include translating one or more user input stimulation zones into a set of electrodes for delivering electrical stimulation therapy to a patient, and a set of parameters such as pulse current amplitudes associated with such electrodes. Programming may further include manipulating the shape and position of the zone, including behaviors of the zone while moving and when colliding with other zones or system interlocks. As the stimulation zone is sized, moved, or shaped, the programmer may automatically compute updated electrode selections and parameters for delivery of stimulation indicated by the stimulation zone. In one example, a user may import an image of the anatomical region in which the leads are implanted and use the imported image as background during programming. A graphical representation of the leads used in programming for the specific region may be over imposed on the image background and manipulated such that the graphical representation of the leads corresponds to the placement of the leads in the region.

Although the disclosure generally refers to implantable stimulators for purposes of illustrations, techniques described in this disclosure also may be used with other types of IMDs, including implantable fluid delivery devices, such as insulin pumps, intra-thecal drug delivery pumps, or other devices that deliver medication or other fluids via one or more fluid delivery elements such as catheters. Such devices may provide fluid delivery therapy for chronic pain, diabetes, or any of a variety of other disorders. In each case, the device may include one or more therapy delivery elements such as one or more catheters implanted within a therapy region. In some cases, a pump may be fully implantable or may be an external device coupled to one or more percutaneously implanted catheters that extend into a therapy region. Accordingly, description of implantable stimulators is provided for purposes of illustration and should not be considered limiting of the techniques as broadly described in this disclosure.

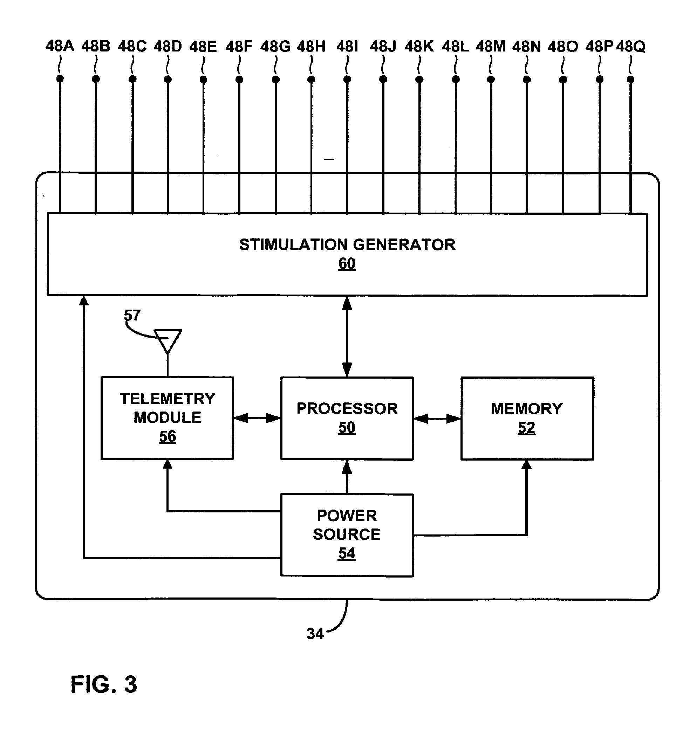

FIG. 3 is a block diagram illustrating various components of an example implantable stimulator 34. Although the components shown in FIG. 3 are described in reference to implantable stimulator 34, the components may also be included within implantable stimulator 4 shown in FIG. 1 and used within system 2. In the example of FIG. 3, implantable stimulator 34 includes processor 50, memory 52, power source 54, telemetry module 56, antenna 57, and a stimulation generator 60. Implantable stimulator 34 is also shown in FIG. 3 coupled to electrodes 48A-Q (collectively "electrodes 48"). Electrodes 48A-48P are implantable and may be deployed on one or more implantable leads. With respect to FIG. 1, lead segments 12A and 12B may carry electrodes 48A-H and electrodes 48I-P, respectively. In some cases, one or more additional electrodes may be located on or within the housing of implantable stimulator 34, e.g., to provide a common or ground electrode or a housing anode. With respect to FIG. 2, leads 32A and 32B may carry electrodes 48A-H and electrodes 48I-P, respectively. In the examples of FIGS. 1 and 2, a lead or lead segment carries eight electrodes to provide an 2x8 electrode configuration (two leads with 8 electrodes each), providing a total of sixteen different electrodes. The leads may be detachable from a housing associated with implantable stimulator 34, or be fixed to such a housing.

In other examples, different electrode configurations comprising a single lead, two leads, three leads, or more may be provided. In addition, electrode counts on leads may vary and may be the same or different from a lead to lead. Examples of other configurations include one lead with eight electrodes (1x8), one lead with 12 electrodes (1x12), one lead with 16 electrodes (1x16), two leads with four electrodes each (2x4), three leads with four electrodes each (3x4), three leads with eight electrodes each (3x8), three leads with four, eight, and four electrodes, respectively (4-8-4), two leads with 12 or 16 electrodes (2x12, 2x16), or other configurations. In some examples, electrode configurations may include percutaneous leads, surgical leads, or segmented leads with different number of electrodes on each segment based on the therapy applied by stimulator 34. Different electrodes are selected to form electrode combinations. Polarities are assigned to the selected electrodes to form electrode configurations.

Electrode 48Q represents one or more electrodes that may be carried on a housing, i.e., can, of implantable stimulator 4. Electrode 48Q may be configured as a regulated or unregulated electrode for use in an electrode configuration with selected regulated and/or unregulated electrodes among electrodes 48A-48P, which may be located on a lead body of one or more leads, as described above. Electrode 48Q may be formed together on a housing that carries the electrode and houses the components of implantable stimulator 4, such as stimulation generator 60, processor 50, memory 52, telemetry module 56, and power source 54.

Housing electrode 48Q may be configured for use as an anode to source current substantially simultaneously with current sourced by one or more other electrodes 48A-48P to form a unipolar or omnipolar electrode arrangement. By way of specific example, in an omnipolar arrangement, electrodes 48A, 48B, and housing electrode 48Q each could be configured for use as anodes. Electrodes 48A, 48B could deliver electrical stimulation current substantially simultaneously with the electrical stimulation current delivered via housing electrode 48Q. In this illustration, one or more cathodes could be formed with other electrodes (e.g., any of electrodes 48C-48P) on the leads to sink current sourced by anodes 48A, 48B and 48Q. Any of a variety of electrode arrangements such as unipolar, bipolar, multipolar, or omnipolar arrangements may be used to deliver stimulation. Accordingly, discussion of particular arrangements are provided for purposes of illustration, which should not be considered limiting of the techniques broadly described in this disclosure.

Memory 52 may store instructions for execution by processor 50, stimulation therapy data, sensor data, and/or other information regarding therapy for patient 6. Processor 50 may control stimulation generator 60 to deliver stimulation according to a selected one or more of a plurality of programs or program groups stored in memory 52. Memory 52 may include any electronic data storage media, such as random access memory (RAM), read-only memory (ROM), electronically-erasable programmable ROM (EEPROM), flash memory, or the like. Memory 52 may store program instructions that, when executed by processor 50, cause the processor to perform various functions ascribed to processor 50 and implantable stimulator 4 in this disclosure.

In accordance with the techniques described in this disclosure, information stored on the memory 52 may include information regarding therapy that the patient 6 had previously received or information regarding a current therapy. Storing such information may be useful for subsequent treatments such that, for example, a clinician may retrieve the stored information to determine the therapy applied to the patient during a previous session, in accordance with this disclosure. The information stored in the memory 52 may be, for example, an image captured and downloaded into the implantable stimulator 34 by a programmer, such as clinician programmer 20 by wireless telemetry. As an example, the image may be obtained during an in-clinic programming session, and may show, for example, lead configuration and placement within a therapy region targeted by one or more leads implanted in the therapy region, in accordance with this disclosure. Images stored in memory 52 may represent different regions to which therapy should be delivered, and may be retrieved by the programmer to combine with a representation of leads or lead groups to effectively deliver therapy in subsequent sessions. As another example, the image may be obtained during the current therapy session. The therapy region could be any of several anatomical regions of patient in which one or more leads may be implanted for delivery of therapy, including the spinal cord, the occipital region, the brain, the pelvic floor, the heart, the gastrointestinal tract, one or more limbs, or the like. In one example, other types of anatomical representations of therapy regions may be utilized, in accordance with the techniques described in this disclosure.

The programmer may obtain the image by capturing a photograph of a hard copy or electronic display presenting an image obtained by a diagnostic imaging device, such as a fluoroscopic imaging or other x-ray imaging device, a magnetic resonance imaging device, an ultrasonic imaging device, electrical impedance topography, or other imaging devices. For example, the programmer may include an integrated digital camera or may be coupled to a digital camera, by a wired or wireless communication medium. Alternatively, the programmer may obtain the image electronically from an imaging device, a network storage server, a removable storage medium such as Flash memory, or other devices. In each case, the image may be stored at least temporarily on the programmer, permitting viewing, manipulation, compression or editing of the image. In some examples, a user may manipulate, compress or edit the image to produce an image suitable or desirable for downloading to the IMD for storage. Also, in some examples, the IMD may store multiple images, e.g., from different regions, different perspectives, or with different views, such as different zoom factors, cropping, spatial resolution, image density resolution or the like. In one example, the IMD may be used to deliver therapy to multiple regions in the patient, and may store images associated with the different therapy regions.

The image may be subsequently retrieved from the IMD, either by a patient programmer or clinician programmer, or both, for any of several reasons such as, for example, later viewing, or subsequent programming and/or therapy delivery to the region associated with the image. In some cases, storing the image in an IMD may permit a clinician to retrieve and upload the image and thereby view the image without the requirement for storage of the image in the clinic or on the programmer. Rather, the clinician may use a different programmer, or the patient may visit a different clinic. In each case, the image may be accessed for review and verification of lead configuration because it is conveniently stored in the IMD, which is ordinarily with the patient. In some cases, the image may alternatively or additionally be stored on a patient programmer, which may be with the patient. However, storing the image in the IMD may ensure that the image may be accessed by an external programmer whenever the patient is present, e.g., whenever the patient visits a clinic for a programming session or other evaluation.

Processor 50 may include one or more microprocessors, digital signal processors (DSPs), application-specific integrated circuits (ASICs), field-programmable gate arrays (FPGAs), or other digital logic circuitry. Processor 50 controls operation of implantable stimulator 34, e.g., controls stimulation generator 60 to deliver stimulation therapy according to a selected program or group of programs retrieved from memory 52. For example, processor 50 may control stimulation generator 60 to deliver electrical signals, e.g., as stimulation pulses or continuous waveforms, with current amplitudes, pulse widths (if applicable), and rates specified by one or more stimulation programs. Processor 50 may also control stimulation generator 60 to selectively deliver the stimulation via subsets of electrodes 48, also referred to as electrode combinations, and with polarities specified by one or more programs.

Upon selection of a particular program group, processor 50 may control stimulation generator 60 to deliver stimulation according to the programs in the groups, e.g., simultaneously or on a time-interleaved basis. A group may include a single program or multiple programs. As mentioned previously, each program may specify a set of stimulation parameters relating to a therapy associated with the program, such as, for example, amplitude, pulse width and pulse rate, if applicable. For a continuous waveform, parameters may include such parameters as amplitude and frequency, for example. In addition, each program may specify a particular electrode combination for delivery of stimulation, and an electrode configuration in terms of the polarities and regulated/unregulated status of the electrodes. The electrode combination may specify particular electrodes in a single array or multiple arrays, and on a single lead or among multiple leads.

Stimulation generator 60 is electrically coupled to electrodes 48A-P via conductors of the respective lead, such as lead 12 in FIG. 1 or leads 32 in FIG 2, in implementations in which electrodes 48A-P are carried by, located on, leads. Stimulation generator 60 may be electrically coupled to one or more housing ("can") electrodes 48Q via an electrical conductor disposed within the housing of implantable stimulator 4 (FIG. 1) or implantable stimulator 34 (FIG 3). A housing electrode 48Q may be configured as a regulated or unregulated electrode to form an electrode configuration in conjunction with one or more of electrodes 48A-48P located on leads of the IMD. Housing electrode 48Q may be configured for use as an anode to source current substantially simultaneously with one or more electrodes, e.g., any of electrodes 48A-48P, on one or more leads configured for use as anodes.

Stimulation generator 60 may include stimulation generation circuitry to generate stimulation pulses or waveforms and circuitry for switching stimulation across different electrode combinations, e.g., in response to control by processor 50. Stimulation generator 60 produces an electrical stimulation signal in accordance with a program based on control signals from processor 50.

For example, stimulation generator 60 may include a charging circuit that selectively applies energy from power source 54 to a capacitor module for generation and delivery of a supply voltage for generation of stimulation signal. In addition to capacitors, the capacitor module may include switches. In this manner, the capacitor module may be configurable, e.g., based on signals from processor 50, to store a desired voltage for delivery of stimulation at a voltage or current amplitude specified by a program. For delivery of stimulation pulses, switches within the capacitor module may control the widths of the pulses based on signals from processor 50.

Stimulation generator 60 may be configured to deliver stimulation using one or more of electrodes 48A-P as stimulation electrodes, e.g., anodes, while substantially simultaneously delivering stimulation using housing electrode 48Q as a stimulation electrode, e.g., anode. The anodes on the lead(s) and the housing may be used to deliver stimulation in conjunction with one or more cathodes on the lead(s). As one illustration, an electrode combination selected for delivery of stimulation current may comprise an anode on the IMD housing, and anode on a lead, and a cathode on the same lead or a different lead. In other examples, the electrode combination may include multiple anodes and/or multiple cathodes on one or more leads in conjunction with at least one anode on the IMD housing.

Telemetry module 56 may include a radio frequency (RF) transceiver to permit bi-directional communication between implantable stimulator 34 and each of clinician programmer 20 and patient programmer 22. In one example, telemetry module 56 may utilize other communication protocols and a corresponding transceiver, for example, a Bluetooth^{®} transceiver for telemetry using the Bluetooth^{®} protocol. Telemetry module 56 may include an antenna 57 that may take on a variety of forms. For example, antenna 57 may be formed by a conductive coil or wire embedded in a housing associated with medical device 4. Alternatively, antenna 57 may be mounted on a circuit board carrying other components of implantable stimulator 34 or take the form of a circuit trace on the circuit board. In this way, telemetry module 56 may permit communication with clinician programmer 20 and patient programmer 22 in FIG 1 or external programmer 40 in FIG. 2, to receive, for example, new programs or program groups, or adjustments to programs or program groups.

Telemetry module 56 may also permit communication with clinician programmer 20 to send, for example, images of therapy regions in the patient so that a programmer may set up and deliver stimulation therapy accordingly. Telemetry module 56 may also receive from programmer 20, for example, combined images representing therapy regions with representation of lead placements within the therapy regions for future access by a programmer, in accordance with this disclosure.

Power source 54 may be a non-rechargeable primary cell battery or a rechargeable battery and may be coupled to power circuitry. However, the disclosure is not limited to examples in which the power source is a battery. In another example, as an example, power source 54 may comprise a supercapacitor. In some examples, power source 54 may be rechargeable via induction or ultrasonic energy transmission, and include an appropriate circuit for recovering transcutaneously received energy. For example, power source 54 may be coupled to a secondary coil and a rectifier circuit for inductive energy transfer. In additional examples, power source 54 may include a small rechargeable circuit and a power generation circuit to produce the operating power. Recharging may be accomplished through proximal inductive interaction between an external charger and an inductive charging coil within stimulator 4. In some examples, power requirements may be small enough to allow stimulator 4 to utilize patient motion at least in part and implement a kinetic energy-scavenging device to trickle charge a rechargeable battery. A voltage regulator may generate one or more regulated voltages using the battery power.

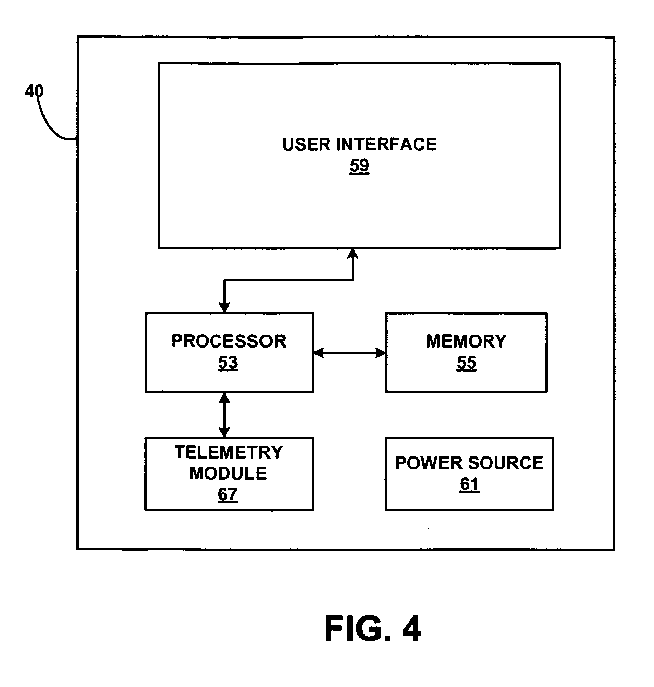

FIG 4 is a functional block diagram illustrating various components of an external programmer 40 for an implantable stimulator 14. Although the components shown in FIG. 4 are described in reference to external programmer 40, the components may also be included within clinician programmer 20 or patient programmer 22 shown in FIG 1. As shown in FIG 4, external programmer 40 includes processor 53, memory 55, telemetry module 67, user interface 59, and power source 61. In general, processor 53 controls user interface 59, stores and retrieves data to and from memory 55, and controls transmission of data with implantable stimulator 34 through telemetry module 67. Processor 53 may take the form of one or more microprocessors, controllers, DSPs, ASICS, FPGAs, or equivalent discrete or integrated logic circuitry. The functions attributed to processor 53 herein may be embodied as software, firmware, hardware or any combination thereof.

Memory 55 may store instructions that cause processor 53 to provide various aspects of the functionality ascribed to external programmer 40 herein. Memory 55 may include any fixed or removable magnetic, optical, or electrical media, such as RAM, ROM, CD-ROM, magnetic disks, EEPROM, or the like. Memory 55 may also include a removable memory portion that may be used to provide memory updates or increases in memory capacities. A removable memory may also allow patient data to be easily transferred to another computing device, or to be removed before programmer 40 is used to program therapy for another patient. Memory 55 may also store information that controls operation of implantable stimulator 4, such as therapy delivery values.

A clinician or patient 36 interacts with user interface 59 in order to, for example, manually select, change or modify programs, e.g., by adjusting voltage or current amplitude, adjusting pulse rate, adjusting pulse width, or selecting different electrode combinations or configurations, and may provide efficacy feedback or view stimulation data. User interface 59 may include a screen and one or more input hard and/or soft key buttons that allow external programmer 40 to receive input from a user. The screen may be a liquid crystal display (LCD), plasma display, dot matrix display, or touch screen. The input buttons may include a touch pad, increase and decrease buttons, emergency shut off button, and other input media needed to control the stimulation therapy.

Using the techniques of this disclosure, a clinician or patient 36 may graphically define one or more desired stimulation regions using interface 59. Using user interface 59, a user may view a combined image that includes an anatomical region of a patient, e.g., in which one or more leads may be implanted, and a graphical layer representing placement of one or more leads implanted in the region. The image of the region may be obtained following lead implant, and the placement of the leads within the implant region may be visible in the image. At the time the image is obtained, the user may add annotations regarding, for example, the placement of the leads, the therapy stimulated by the leads, and the patient. The image may be an image obtained via any of a variety of imaging modalities such as a fluroscope or other x-ray imaging device, an ultrasound imaging device, a magnetic resonance imaging device, electrical impedance tomography, or other imaging devices. The image may be obtained during a previous therapy session or during the current therapy session, stored in the IMD, and subsequently retrieved by a programmer. In one example, the image may be stored in the patient programmer. In another example, the image may be stored on a local or network drive, or a removable memory device.

The graphical layer may be used to place a graphical representation of the leads over the leads in the image, and the user may manipulate the position and curvature of the graphical representation of the leads to match that of the implanted leads. The graphical representation of the lead may be used in a programming procedure to program a desired electrical stimulation therapy more effectively and accurately. For example, the graphical representation of the lead, which is combined or overlaid with the image of the anatomical region, may be used to select particular electrodes forming an electrode combination, and assign current or voltage amplitudes to the electrodes. In some cases, the programmer may be configured to permit a user to define stimulation zone with respect to a set of electrodes depicted by the graphical representation of the lead, and permit the user to manipulate the stimulation zone, e.g., by sizing, shaping or repositioning the zone. As the zone is manipulated, the programmer may automatically update the selection of electrodes associated with the zone, and adjust stimulation intensity (e.g., pulse current or pulse voltage) representing stimulation intensity contributions of the electrodes (e.g., as source or sink electrodes) in forming a stimulation field according to the stimulation zone. The combined image, including the image of the anatomical region and the graphical image of the lead representation, may be stored in the programmer or in the IMD or on a removable storage device, e.g., for access by different programmers in future clinical sessions to ensure regions defined by the images are available for later evaluation.

Telemetry module 67 allows the transfer of data to and from stimulator 34. Telemetry module 67 may communicate automatically with stimulator 34 at a scheduled time or when the telemetry module detects the proximity of the stimulator. Alternatively, telemetry module 67 may communicate with stimulator 34 when signaled by a user through user interface 59. To support RF communication, telemetry module 44 may include appropriate electronic components, such as amplifiers, filters, mixers, encoders, decoders, and the like. In other examples, telemetry module 67 may employ other communication standards such as, for example, Bluetooth^{®} and telemetry module 67 may include the appropriate Bluetooth^{®} components.

Programmer 40 may communicate wirelessly with implantable stimulator 34 using, for example, RF communication or proximal inductive interaction or other communication standards such as, for example, Bluetooth^{®}. This wireless communication is possible through the use of telemetry module 67 which may be coupled to an internal antenna or an external antenna. Telemetry module 67 may be similar to telemetry module 57 of implantable stimulator 34. In accordance with this disclosure, programmer 40 may communicate with stimulator 34, via telemetry module 56 to retrieve information such as, for example, images of regions with lead placements and previously delivered therapy, which may be stored in memory 52 for viewing and/or manipulation by a user via user interface 59.

Programmer 40 may also be configured to communicate with another computing device via wireless communication techniques, or direct communication through a wired, e.g., network, connection. Examples of local wireless communication techniques that may be employed to facilitate communication between programmer 24 and another computing device include RF communication based on the 802.11 or Bluetooth^{®} specification sets, infrared communication, e.g., based on the IrDA standard.

Power source 61 delivers operating power to the components of programmer 40. Power source 61 may be a rechargeable battery, such as a lithium ion or nickel metal hydride battery. Other rechargeable or conventional batteries may also be used. In some cases, external programmer 40 may be used when coupled to an alternating current (AC) outlet, i.e., AC line power, either directly or via an AC/DC adapter. Power source 61 may include circuitry to monitor power remaining within a battery. In this manner, user interface 59 may provide a current battery level indicator or low battery level indicator when the battery needs to be replaced or recharged. In some cases, power source 61 may be capable of estimating the remaining time of operation using the current battery.

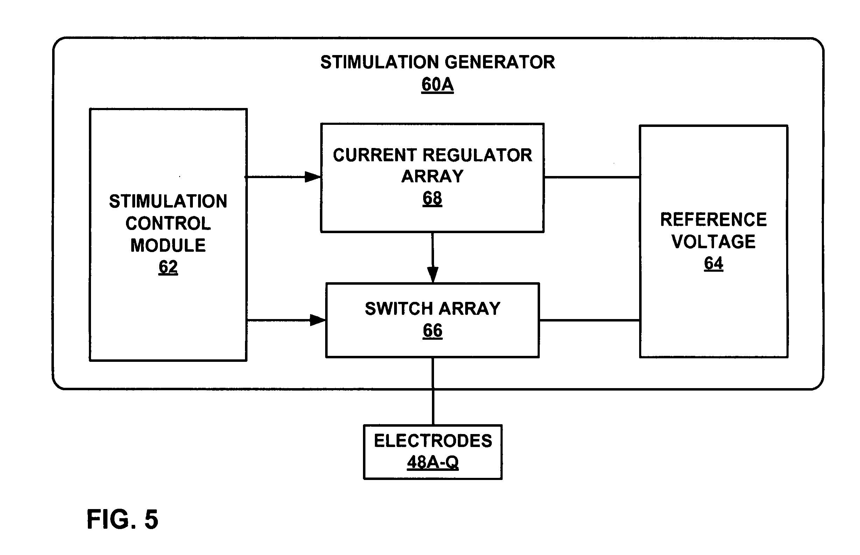

FIG. 5 is a block diagram illustrating various components of an example stimulation generator 60A. Stimulation generator 60A may be used with an implantable stimulator, e.g., to perform the functions of stimulation generator 60 as described with reference to FIGS. 1-3. Although described with respect to implantable stimulator 4, stimulation generator 60A may also be used for implantable stimulator 34, or other types of stimulators. In the example of FIG 5, stimulation generator 60A is selectively, e.g., based on a signal from processor 50 (FIG 3), configured to deliver constant current stimulation pulses to patient 6 via various electrode combinations. However, the disclosure is not limited to examples in which regulated current pulses are delivered. In other examples, stimulation generator 60A may provide continuous, regulated current waveforms, rather than regulated current pulses. In still other examples, stimulation generator 60A may deliver combinations of continuous waveforms and pulses, or selectively deliver either continuous waveforms or pulses. Stimulation generator 60A may generate either constant current-based or constant voltage-based stimulation in the form of pulses or continuous waveforms. In yet other examples, stimulation generator 60A may user a voltage regulator instead of a current regulator.

In the example illustrated in FIG 5, stimulation generator 60A includes stimulation control module 62, reference voltage source 64, switch array 66, and current regulator array 68. Reference voltage source 64 may provide operating power to current regulator array 68, and may include a regulated voltage that sets the level of the reference voltage. As shown in FIG 5, reference voltage source 64 may be coupled to provide operating power for the current regulator array 68 and provide a reference voltage for connection to electrodes 48A-48Q for an unregulated mode of electrode operation. In other examples, however, the voltage level of the reference voltage and the operating voltage level provided to regulated current source array 68 may be different.

Stimulation control module 62 forms a stimulation controller that controls switch array 66 and current regulator array 68 to deliver stimulation via electrodes 48A-48Q. Stimulation control module 62 may include one or more microprocessors, microcontrollers, digital signal processors (DSPs), application-specific integrated circuits (ASICs), field-programmable gate arrays (FPGAs), or other integrated or discrete logic circuitry. In operation, stimulation control module 62 may control delivery of electrical stimulation according to one or more programs that may specify stimulation parameters such as electrode combination, electrode polarity, stimulation current amplitude, pulse rate, and/or pulse width as well as the percentage of source current distributed among or contributed by a housing anode and one or more lead anodes on one or more leads, and the percentage of sink current sunk by one or more cathodes. Programs may be defined by a user via an external controller and transferred to an implantable stimulator 4 or 34 for use by stimulation control module 62.

Current regulator array 68 includes a plurality of regulated current sources or sinks. Again, a current regulator may function as either a current source or sink, or be selectively configured to operate as either a source or a sink. For convenience, however, the term "current regulator" may be used in some instances to refer to either a source or sink. Hence, each of the current regulators in current regulator array 68 may operate as a regulated current source that delivers stimulation via a corresponding one of electrodes 48A-Q or a regulated current sink that receives current from a corresponding one of electrodes 48A-Q, where electrodes 48A-48Q may be provided on leads, on a stimulator housing, on a leadless stimulator, or in other arrangements. In general, electrodes 48A-48Q may be referred to below as electrodes 48 for conciseness.

Each switch of switch array 66 couples a corresponding one of electrodes 48 to either a corresponding bidirectional current regulator of current regulator array 68 or to reference voltage 64. In some examples, stimulation control module 62 selectively opens and closes switches in switch array 66 to configure a housing electrode, e.g., electrode 48Q, and one or more of electrodes 48A-48P on one or more leads as regulated electrodes by connection to regulated current sources or sinks in current regulator array 68. In other examples, stimulation control module 62 may selectively open and close switches in switch array 66 to configure either the housing electrode, e.g., electrode 48Q, or an electrode on the lead as an unregulated electrode by connection to reference voltage 64. In addition, stimulation control module 62 may selectively control individual regulated current sources or sinks in current regulator array 68 to deliver stimulation current pulses to the selected electrodes.

Reference voltage 64 may be a high or low voltage supplied by a regulated power source, depending on whether an electrode is programmed to be an unregulated source (high voltage rail) or unregulated sink (low voltage rail). Hence, reference voltage 64 may produce high and low reference voltages for selective coupling to unregulated, reference electrodes as needed given the selected electrode configuration. A regulated power source may produce one or more regulated voltage levels for use as reference voltage 64 and for use as a power rail for current regulator array 68. Again, although the same reference voltage 64 is coupled to current regulator array 68 in FIG. 5, different voltage levels could be used for the reference voltage coupled to switch array 66 and the operating voltage level provided to the regulated current source array. A regulated power source may generate the regulated voltages from voltages provided by a power source 54 (FIG. 3), such as a battery.

Stimulation control module 62 controls the operation of switch array 66 to produce electrode configurations defined by different stimulation programs. In some cases, the switches of switch array 66 may be metal-oxide-semiconductor field-effect-transistors (MOSFETs) or other circuit components used for switching electronic signals. The switches of switch array 66 may be designed to carry an amount of unregulated current that may be coupled to a corresponding electrode through an unregulated current path associated with reference voltage 64. As previously described, in some examples, two or more regulated stimulation electrodes 48 may be intentionally programmed to deliver different amounts of current such that the regulated electrodes produce an unbalanced current distribution.

To provide individual control of electrodes 48 as either regulated electrodes or as unregulated, reference electrodes, stimulation control module 62 controls operation of switch array 66, and current regulator array 68. When stimulation is delivered to patient 6, for the example of current pulses, stimulation control module 62 controls switch array 66 to couple selected stimulation electrodes for a desired electrode combination to respective current regulators of current regulator array 68 or to reference voltage 64, as needed. Stimulation control module 62 controls the regulated bidirectional current sources of current regulator array 68 coupled to regulated electrodes to source or sink specified amounts of current. For example, stimulation control module 62 may control selected current sources or sinks to on a pulse-by-pulse basis to deliver current pulses to corresponding electrodes.

Stimulation control module 62 also deactivates the regulated bidirectional current regulators of current regulator array 68 tied to inactive electrodes, i.e., electrodes that are not active as regulated electrodes in a given electrode configuration. Each regulated bidirectional current regulator of current regulator array 68 may include an internal enable switch controlled by stimulation control module 62 that disconnects regulated power source 64 from the current regulator or otherwise disables the current source when the corresponding electrode is not used as a regulated electrode to deliver stimulation.

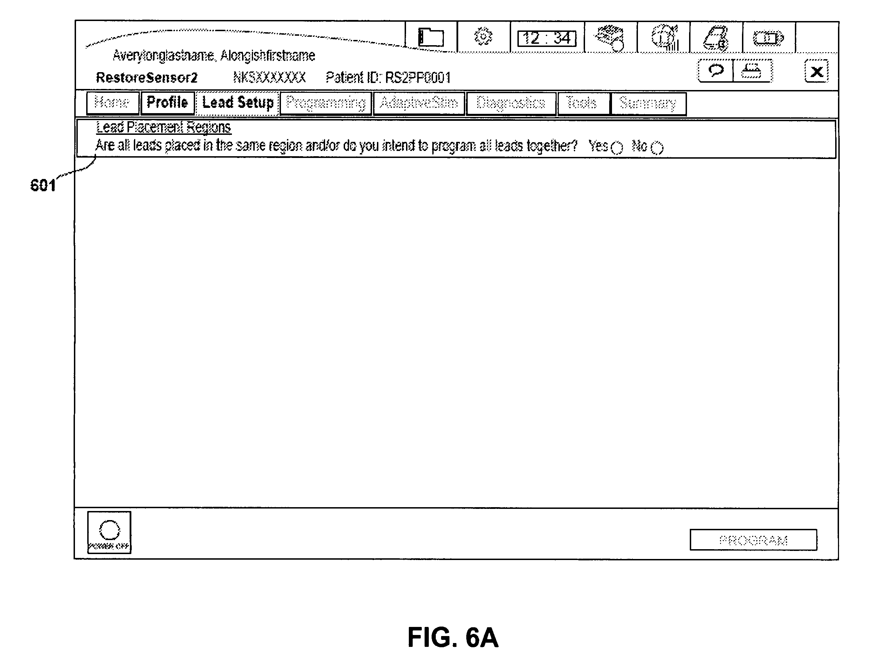

FIGS. 6A-6L illustrate an exemplary sequence of programmer screens, in accordance with this disclosure. FIGS. 6A-6L each show an exemplary screen shot 600 of a display on the user interface 59 on a programmer 40 as described in this disclosure. In one example, programmer 40 may be a clinician programmer. FIGS. 6A-6L are discussed in the context of a patient receiving spinal cord stimulation therapy as an illustrative example. A programmer, e.g., programmer 40, may receive user input via the user interface 59 to set up leads and parameters that define one or more stimulation programs for delivering therapy to a patient. A stimulation program may be delivered by an implantable stimulator individually or in combination with other programs, e.g., on a time-interleaved basis. A program may define an electrode combination, including a selected set of electrodes for delivery of stimulation and polarities of such electrodes, pulse current or pulse voltage amplitudes delivered by respective electrodes, pulse width and pulse rate. The user may set up a profile for the session and the patient, and proceed to configure placement of the leads.

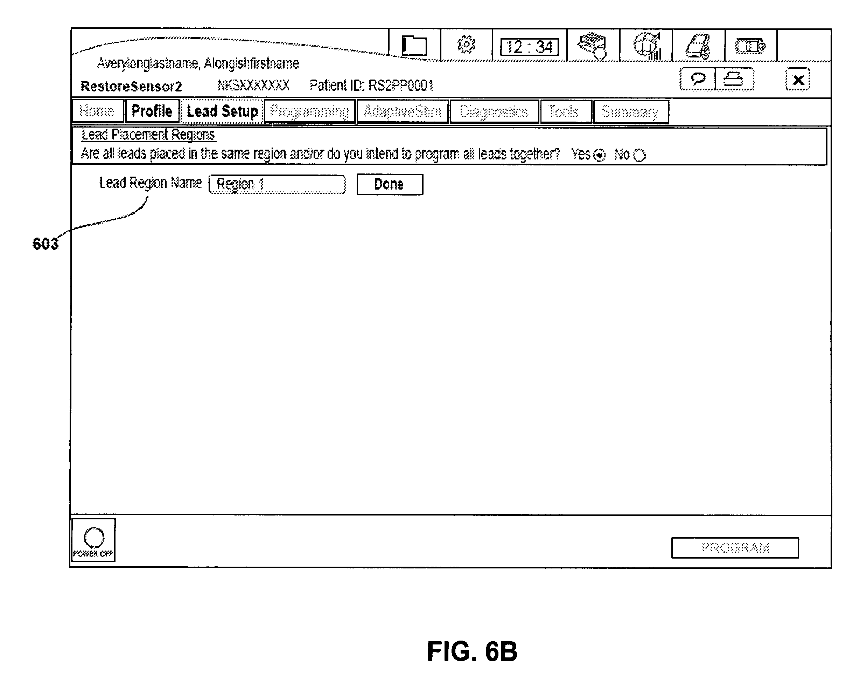

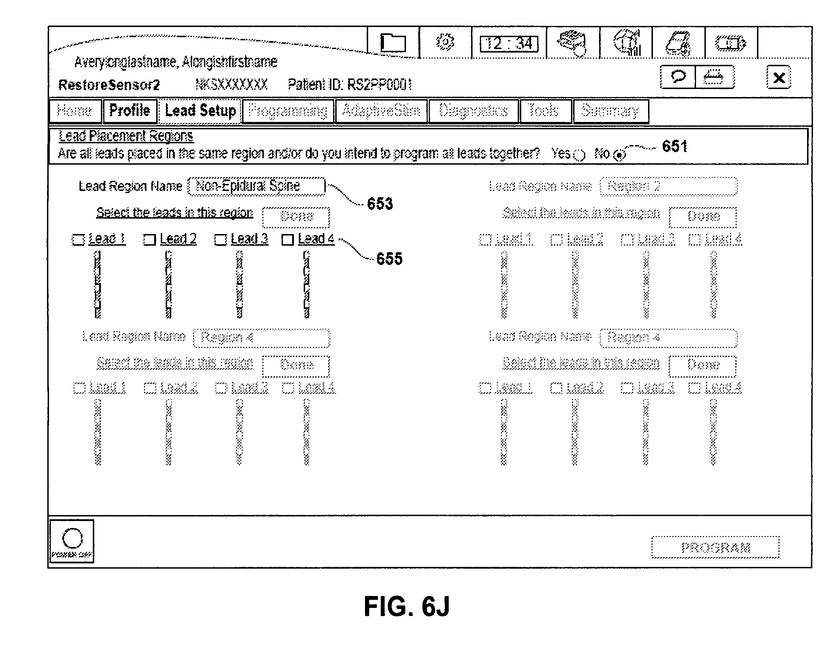

To provide an accurate representation of implanted leads for spinal cord stimulation, the user may select which leads will be programmed with therapy. In this example, there are up to 4 leads from which a user may select. However, in other examples there may be more or less leads available to the user from which to select. FIG. 6A illustrates a lead set up screen where the user is prompted to indicate whether all the leads, for the given number of leads implanted or being trialed, will be placed in the same region and/or programmed together by indicating "Yes" or "No" (601). If the user selects that all the leads will be placed in the same region and/or programmed together, i.e., the user selects "Yes," the lead set up screen may prompt the user to enter a name for the lead region to which the leads are assigned (603), as shown in FIG. 6B. Otherwise, as shown in FIG. 6J, the user may respond with "No" (651), indicating that leads will be assigned to different lead regions and/or programmed separately. As a result, the lead set up screen may prompt the user to enter a name for each lead region (653) (in this example, 4 regions, but may be more or less regions) and to select the leads (655) that will be programmed for each of the lead regions (in this example, 4 leads per region, but may be more or less leads). A lead region may be, for example, such entity as "lumbar spine" or "epidural thoracic." The user may assign implanted leads to one or more regions, where the regions represent the anatomical implant location of the leads associated with the regions. In one example, where different leads are selected for different regions, the user may view and/or program each region where only the leads associated with a selected region are viewed and/or programmed. In one example, the user may assign the case electrodes to one or more regions, either alone or in combination with the implanted leads.

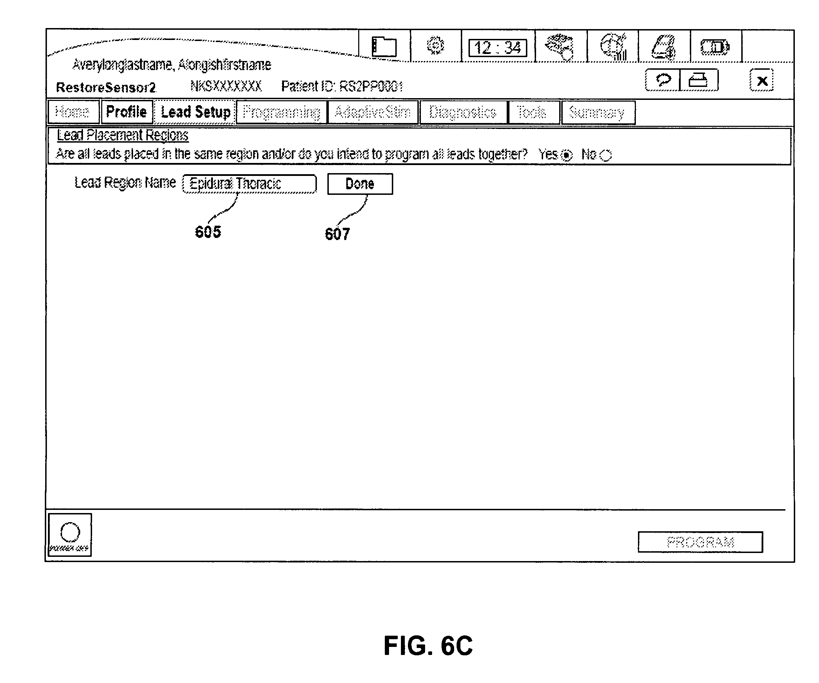

After the leads are organized, the region where they are implanted may be named (605 and 653) by the user (epidural thoracic, etc.), as shown in FIG. 6C and 6J. When the user enters a name for the lead region or selects one from a checklist or drop-down menu, the "Done" button (607) may be highlighted allowing the user to select it and go on to the next screen, as shown in FIG. 6C. Referring to FIG. 6J, when the user enters a name for a lead region and selects the leads for that region, the "Done" button may be highlighted (not shown highlighted as no leads have been selected) allowing the user to move on to the next lead region to name it and select the corresponding leads, and so forth, until all the lead regions (in this example 4 lead regions, which may be more or less in other examples) and named and corresponding leads are selected for each lead region.

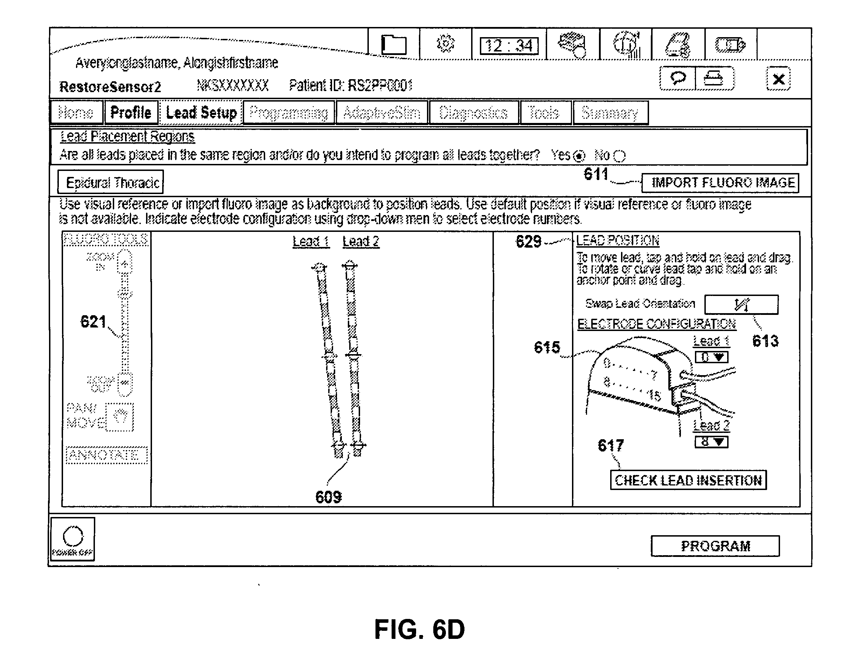

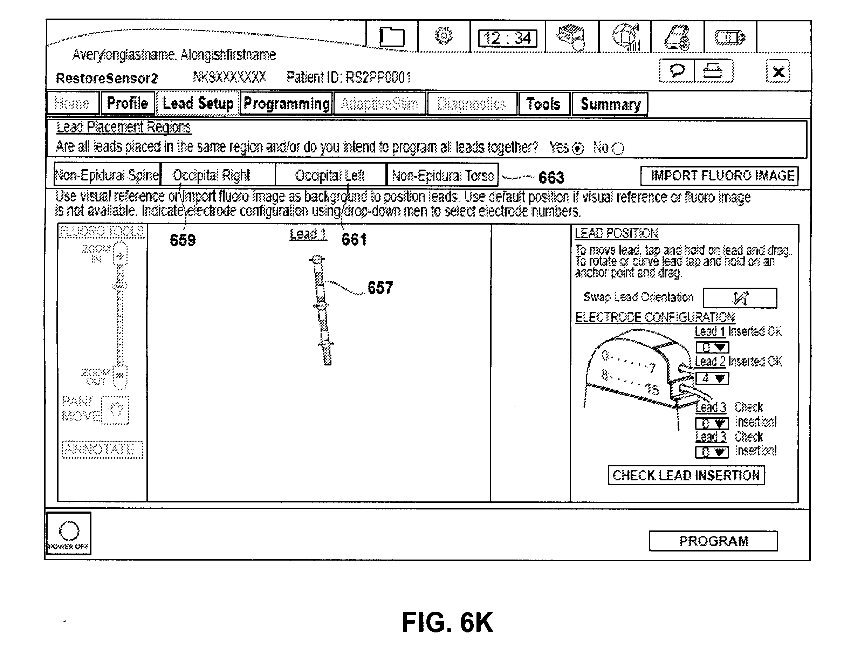

Once the lead regions are named, a representation of the leads to be programmed for that region may be shown on the screen. Referring to FIG. 6D, the representation of two leads (609), each with 8 electrodes may be shown on the screen, because the user selected to program them all together (in this example two leads). Alternatively, referring to FIG. 6K, if multiple lead regions are programmed, representation of the leads (657) for each lead region may be shown on the screen. In the multiple lead region scenario of FIG. 6K, for example, for the lead region named "Non-Epidural Spine," the user selected lead 1, which has four electrodes in this example, to be programmed for this lead region, and therefore, only lead 1 (657) is displayed on the screen for the lead region "Non-Epidural Spine." Each of the remaining lead regions, e.g., "Occipital Right" (659), "Occipital Left" (661), and "Non-Epidural Torso" (663), has its own tab, and when the tab for a lead region is selected, the screen will display the leads the user selected (in screen of FIG. 6J) for that region. The discussion at this point will deal with the screens for the example where all leads were selected to be programmed for one region and together, but it should be noted that the same discussion can be applied to the case of multiple regions.

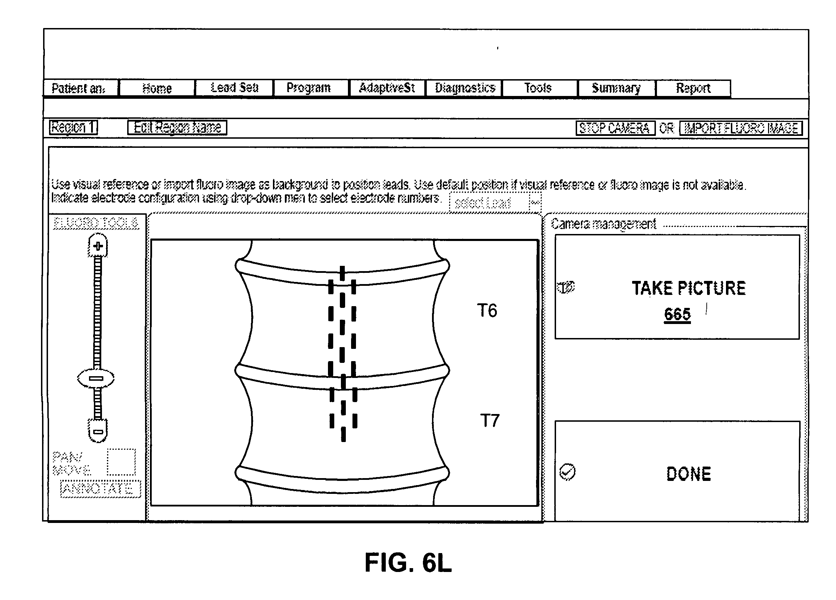

Referring again to FIG. 6D, the graphical representation of the leads (609) may be displayed on the screen, and the user may be provided with more options from which to select. The user may be presented with an option to import an image for the lead region being programmed (611). The image may be for example a fluoroscopic image of the region in which therapy is to be applied. A button (611) labeled "Import Fluoro Image" appears on the display as shown in FIG. 6D, which the user may select if the user opts to import a fluoroscopic image of the leads assigned to the region for which therapy is being programmed. In another example, the display may also have a "Start Camera" button (not shown), which may, upon selection by the user, display an image acquired by the camera, which the user may select to use as the background image by clicking a button (665) labeled "Take Picture," as shown in FIG. 6L, as an alternative for importing a previously-acquired image. Therefore, a user may have an option to either use the camera or import a previously-acquired image, as shown in FIG. 6L. One or more examples of acquiring and storing a fluoroscopic image of a therapy region in a patient may be described in United States Patent Application, to Davis et al., titled "STORING IMAGE OF THERAPY REGION IN IMPLANTABLE MEDICAL DEVICE", published as US 2011-093047.

The display screen may also present options to the user to manipulate the positions or the representation of the leads 609. In the example of FIG. 6D, the user may move a lead by tapping and holding on the lead and dragging it, or the user may rotate or curve a lead by tapping and holding on an anchor point and dragging as indicated by the instructions (629) provided to the user on the screen. The user may also swap the lead orientation by selecting the corresponding function button (613). The user may also manipulate the leads further by bending, resizing, and shaping the graphical representation of the leads or portions of the leads to achieve a better match between the graphical leads and the image of the leads in the anatomical background image. In one example, the user may select starting and ending points of the leads in the image (e.g., to match distal and proximal electrode locations in the image), and the programmer may automatically draw the graphical representation of the leads between the two points. The display may also show the electrode configuration (615), showing how the leads are inserted. In one example, an option (617) may be displayed to "Check Lead Insertion" to check that the leads are inserted correctly. The display may also present the user with the ability to manipulate the fluoroscopic image, as shown on the left side of the screen. However, the fluoroscopic image tools may not be highlighted until a fluoroscopic image is actually imported (see FIG. 6D, where user has not imported an image yet, and the fluoroscopic image tools are grayed out (621)).

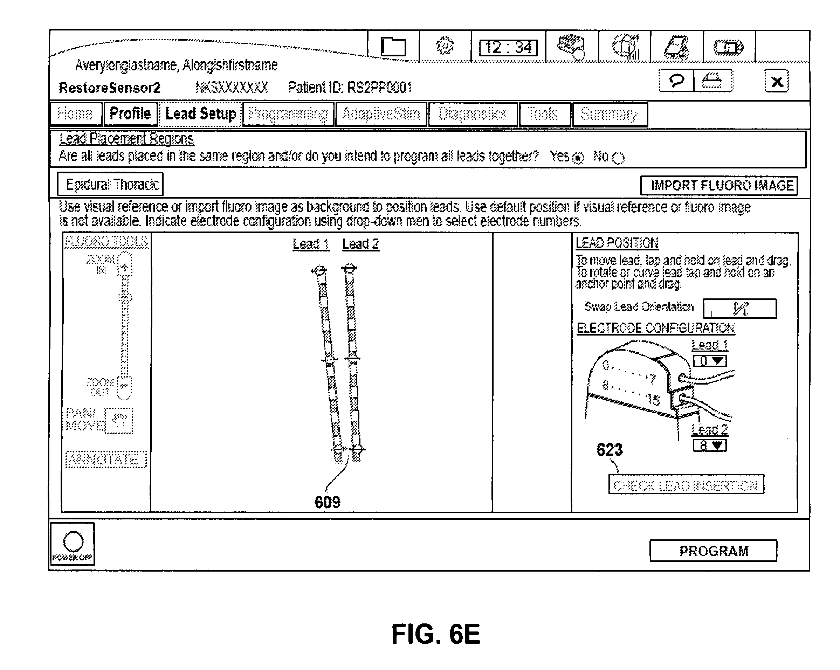

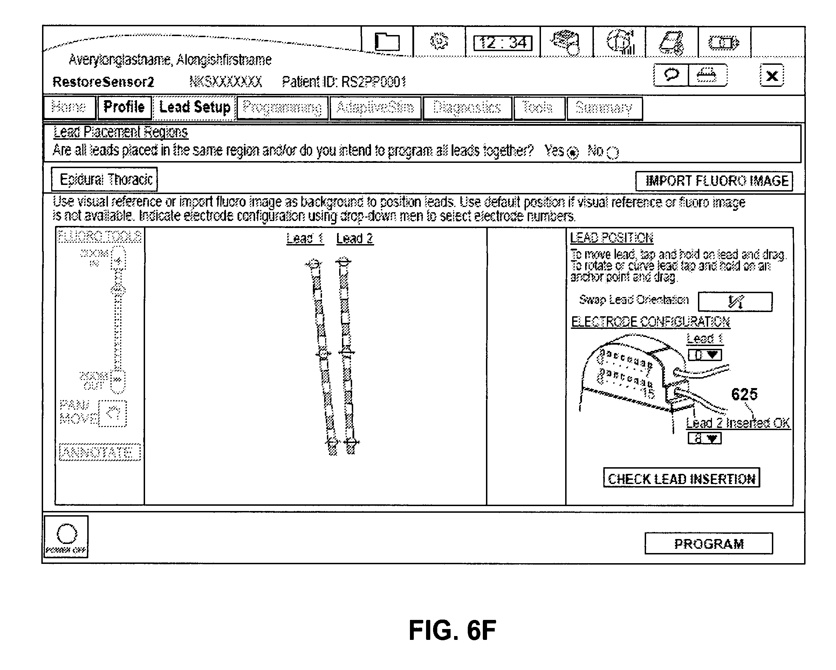

Referring to FIG. 6E, the user may select to check the lead insertion by selecting the "Check Lead Insertion" button (623) when it is available (i.e., not grayed out). For example, the "Check Lead Insertion" button (623) may be unavailable if certain information is missing, e.g., assignment of electrode numbers to leads, or if lead insertion was previously checked, no problems were detected, and no other changes were detected. In one example, lead insertion may allow the user to ensure that the lead is correctly inserted into the header. The lead insertion check may be, for example, an impedance measurement between pairs of electrodes along the lead (e.g., impedance from electrode 0 to electrode 1, impedance from electrode 1 to electrode 2, etc.). The "Check Lead Insertion" button (623) may be grayed out during the measurement. Referring to FIG. 6F, the display may indicate to the user the results of checking the insertion of the leads, and if, as in this case, there are no issues with the insertion of either lead, next to each lead under "Lead Configuration" on the right side of the screen the text "Inserted OK" (625) may be displayed. In one example, there may be issues with lead insertion, and the user interface may display "NOT OK," which may signal to the user to manipulate the lead or remove the lead and reinsert it into the header and check again. In one example, the lead insertion check may result in a "NOT OK" if certain issues exist such as, for example, no lead is present (e.g., the impedance for all electrodes is out of range), the lead partially inserted (e.g., the impedance of only a subset of electrodes is in range, indicating partial insertion and the impedance of the remaining leads is out of range), the lead is fully inserted but poor connections exist (e.g., the lead is fully inserted, but the impedance of one or more electrodes is out of range), possible fluid in connector (e.g., the impedance of one or more electrodes is low, indicating shorts exist).

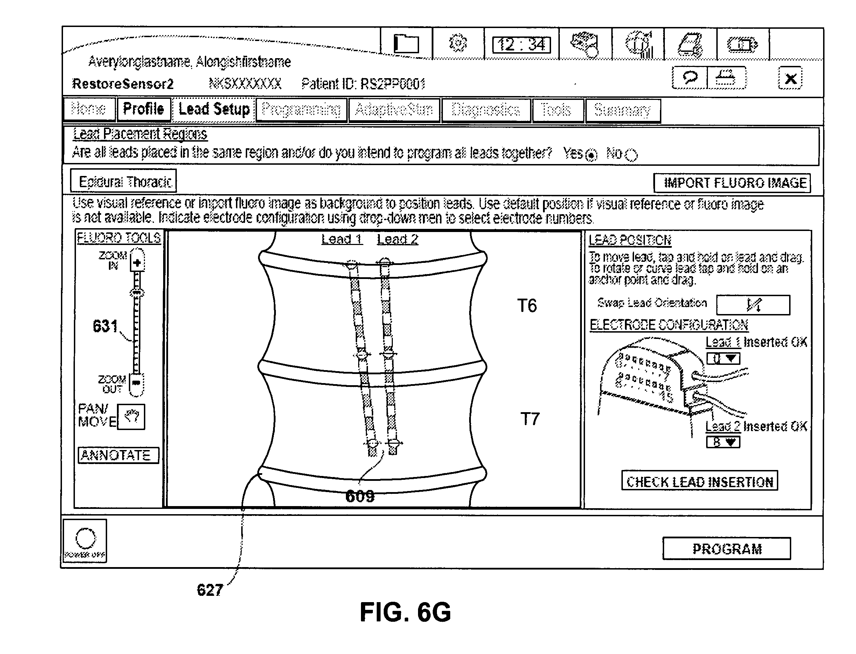

Referring to FIG. 6G, the user may then select to import a fluoroscopic image, and the image (627) may be imported. The representation of the leads (629) may be superimposed on the fluoroscopic image (627) of the leads. While it is not shown, a similar screen may be displayed for each of the regions when multiple regions are defined, and the user may import a fluoroscopic image to one or more regions, by clicking on the tab for the region (see FIG. 6K) and clicking on the "Import Fluoro Image" for that region.

In one example, the representation of the leads (609) may not be initially aligned with the leads in the fluoroscopic image (629). To get the representation of the leads (609) to more accurately align with leads in the fluoroscopic image (627), the representation of the leads (609) may be scaled, stretched, bent, moved, or rotated to match the actual leads shown in the background imported image. In some examples, the leads may have curvature added to them to match the imported fluoroscopic image. For example, by moving selected anchor points along the lead, the user may move a portion of the lead relative to other portions of the lead to introduce a desired bend, such as a bend that more precisely matches a bend in the actual implanted lead shown in the background image. In addition, the fluoroscopic image (627) may be moved, zoomed, or manipulated to match a generic drawing of the lead, and that can be done once the image is imported and the "fluoro tools" (631) is highlighted as shown in FIG. 6G. The fluoroscopic image (627) may also be annotated, e.g., by text notes, by clicking the "Annotate" button (633). As can be seen in the example image in FIG. 6G, the region in this image includes the T6, T7 vertebrae. Each of leads 1 and 2 are 8-electrode leads. As shown in FIG. 6J and 6K, however, the leads may be 4-electrode leads, or other n-electrode leads in other examples. In one example, when the user selects one of the regions, the image for that region and the graphical representation of the leads assigned to that region may be displayed on the user interface.

In one example, the user interface may display the image and graphical representation of leads in 2D. In this example, the user interface may display the same region and the corresponding graphical representation of leads implanted in the region in 2D from different perspectives corresponding to different angles or cross sections, as the implanted leads may curve in more than one direction spatially, and allow the user to manipulate the graphical representation of leads for each perspective. In another example, the user interface may display the image and graphical representation of leads in 3D. In this example, the user interface may display the region and the corresponding graphical representation of leads implanted in the region in 3D, and may allow the user to rotate and manipulate the image and the graphical representation in all directions.

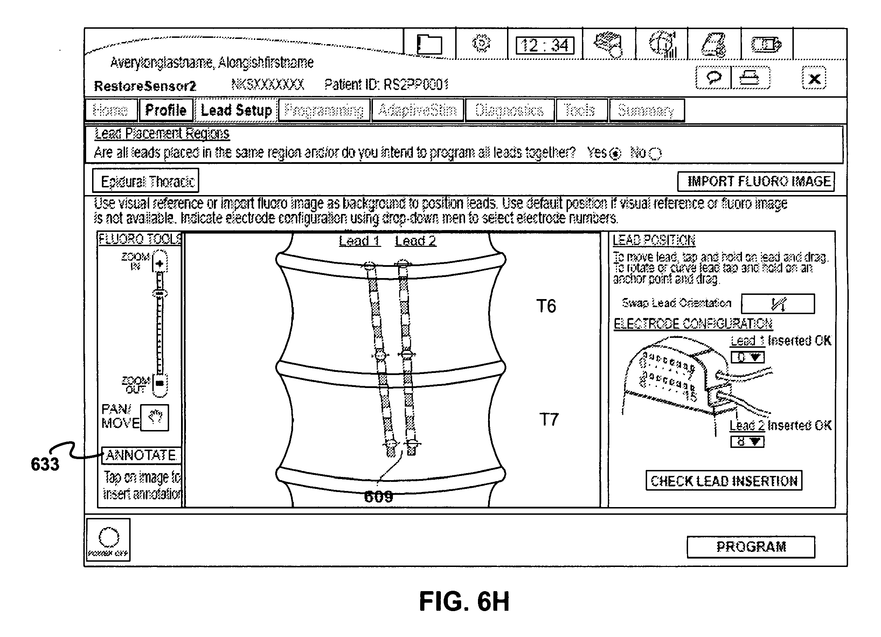

Referring to FIG. 6H, the user may select to annotate the fluoroscopic image by clicking the button (633) "Annotate." The display may then instruct the user to annotate the image by "tapping on image to insert annotation." The user may manipulate the fluoroscopic image (627) and the representation of the leads (609) so that they align. The user may then scale fluoroscopic image (627) and the representation of the leads (609) together or separately. To more easily view the leads, the leads may be drawn as transparent with color outlines. The transparency and color outline allow the user to more easily align with the underlying fluoroscopic image layer.

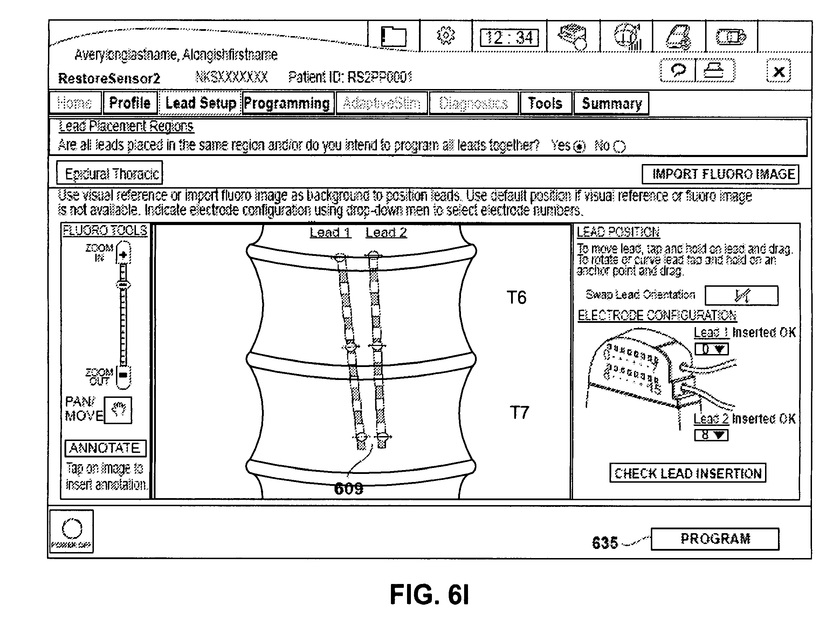

After the leads and fluoroscopic image have been manipulated to match for a given region, the process may be repeated for all lead regions (if multiple regions are programmed). The fluoroscopic image may be compressed and stored in the device, by selecting the button (635) labeled "Program" on the screen, as shown in FIG. 6I. Selecting "program" may program all the changes to the device. The display may also present the user with other options like "Tools" and "Summary" in addition to "Programming." Subsequently, the creation of therapy may occur (per lead region) on the final lead representation of the leads with the fluoroscopic image as background.

Each process described for the example of assigning all the leads to one image, may be used with each of the different regions (for example, the four as defined in FIG. 6K). When multiple regions are defined, multiple fluoroscopic images may be linked to corresponding multiple lead electrode images and locations, and attributes related to the leads and the images may be linked thereto, and the combination of the images, leads, and attributes may be programmed and stored on medical devices, using the techniques described herein. Each of the images may be associated with an anatomical region, or with different perspectives of the same region, for example. Additionally, a lead may be programmed to provide therapy associated with more than one region. In one example, the user may assign and program the case electrodes for certain anatomical regions in the same manner the user assigns and programs implanted leads.

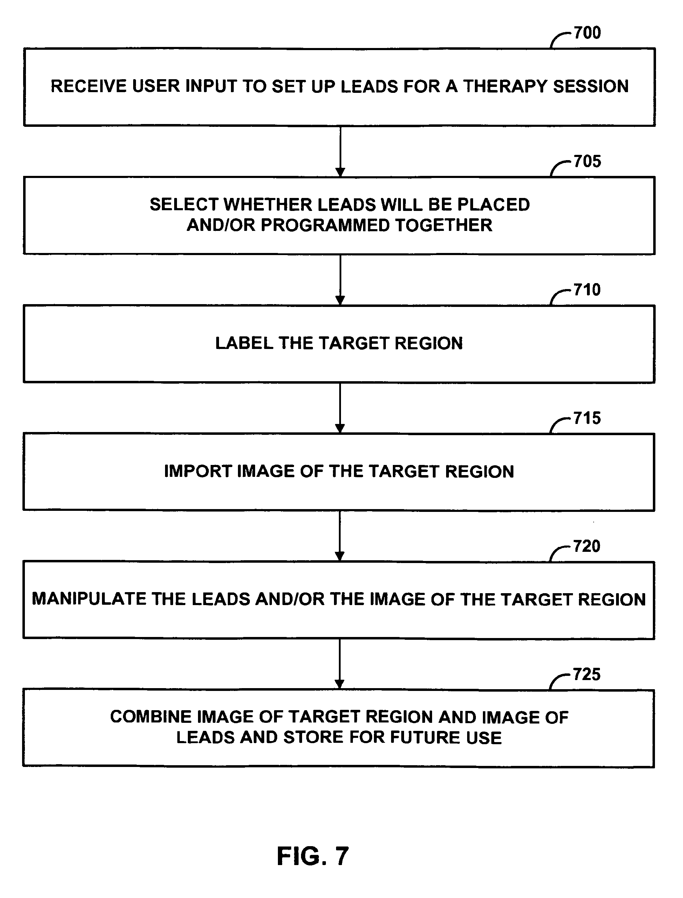

FIG. 7 is a flow diagram illustrating exemplary operation of a programmer to create, assign, and manipulate leads in different anatomical regions in a patient, in accordance with this disclosure. A programmer, e.g., programmer 40, receives user input via the user interface 59 to set up leads (700) for use in defining one or more stimulation programs. A stimulation program may be delivered by an implantable stimulator individually or in combination with other programs, e.g., on a time-interleaved basis. A program may define an electrode combination, including a selected set of electrodes for delivery of stimulation and polarities of such electrodes, pulse current or pulse voltage amplitudes delivered by respective electrodes, pulse width and pulse rate. The user may set up a profile for the session and the patient, and proceed to configure placement of the leads. The user interface may show a screen as shown in FIG. 6A, and may inquire as to whether all the leads will be placed in the same region and/or programmed together.

The user may then select whether the leads will all be placed in the same region and/or programmed together (705). The programmer will then indicate that a lead region is to be created, if the user selects to have all leads placed in the same region, as shown in FIG. 6B. Alternatively, if the user selects NO in response to whether all leads will be placed in the same region, the programmer will display multiple lead regions, as shown in FIG. 6J. In one example, when the user indicates a selection for multiple regions, the programmer may display a selection for the user to indicate the number of lead regions, from which the user may select two or more regions. In the example of FIG. 6J, there may be four regions to define for the current therapy session for the specified user. In another example, the programmer may automatically show the regions associated with the current patient, and the user may be able to change the number of regions if a therapy region is no longer needed or to add a new region.

As a result, in either case, at least one region is created, where lead regions may correspond to anatomical regions, where each region is to receive stimulation therapy corresponding to a symptom, disorder or disease or other problem associated with the specified region for the specified patient. For each lead region, the user may assign leads, which may be chronically implanted or percutaneously implanted for testing before chronic implantation, to deliver therapy to the corresponding region. For each group of one or more leads assigned to a lead region, the user may select which leads will be programmed together and which will be programmed separately. For example, for one region, the most effective therapy may be to group all the leads together and program them to deliver therapy as a group. For another region, the most effective therapy may be to group only a portion of the leads and program them together, and program the rest of the leads either separately or as a separate group. For yet another region, the most effective therapy may be to program each lead separately.

The user may then label or name the region (710). The regions may be for example labeled according to the anatomical name of the region, such as, for example, "lumbar spine" or "cervical spine," as shown in FIG. 6C and FIG. 6K. The regions may also be named by the user using text entry, or may be selected from a drop down menu that contains generic names for regions (e.g., region 1, region 2, etc.), default names associated with commonly targeted regions e.g., lumbar spine, cervical spine, epidural thoracic, non-epidural spine, occipital left, occipital right, non-epidural torso, or the like), or regions associated with the patient receiving the treatment where he had previously received treatment. In another example, the names of the regions may be auto-populated based on entries previously made on the programmer or for the patient.

A representation of the leads may be displayed on the interface, as shown in FIG. 6D, and the user may be given the option to import a stored or captured image representing the region where therapy is to be delivered. Alternatively, if the user elects to not import an image of the region or if such an image is not available, the leads may be configured using a default position/placement. In an example, a default position for the leads may be the vertical position for a region that the user may leave unnamed, for example, if the user elects to name a region "Region 1," the leads may be displayed vertically by default. In another example, if the user elects to name the region, depending on the name, the default position for the leads may be a typical lead positioning for delivering therapy to that region, but can be altered by the user. The default position/placement of the leads may be useful in application where quick programming is desirable, for example, in an operating room. The user may also be capable of checking lead insertion to ensure availability of the leads before proceeding with therapy, as shown in FIG. 6E. The interface may then indicate the results of checking the lead insertion. If there are no issues with the lead insertion, the user interface may indicate that the insertion of each lead is OK, as shown in FIG. 6F. If, for example, there are problems with lead insertion, i.e., the insertion of the leads is not OK (e.g., no lead is inserted in the header, the lead is partially inserted, lead fully inserted by poor connections exist, or possible fluid in connector), the interface may indicate to the user that the leads are not correctly inserted, and may display instructions on how to correct the issue, e.g., re-insert the lead, confirm the correct model numbers for the leads, or the like.

The user may opt to import an image representing the region to which therapy will be applied (715). The image of a region may be a fluoroscopic image of the region, as shown in FIG. 6G. In other examples, the image of the region may be a graphical representation of the region, e.g., an anatomical image of the spinal cord. Importing an image may also be done by acquiring the image at the time of the programming, with a camera on or connected to the programmer, and may appear right away on the screen, as shown in FIG. 6L. In some examples, the image may be a video image of the region or a series of still images showing change over time. Each region defined by the user may be represented by a separate image. The image for each region may be an image captured by the current user of the programmer, may be acquired/captured right at that time with a camera on or connected to the programmer, or may be a stored image, which had been captured during a previous session and stored in the implanted medical device. For example, during a previous session, an image of the lead placement in the region receiving therapy may have been obtained by the programmer, manipulated by the user, and downloaded into the implanted device for subsequent treatments. The manipulation may involve, for example, compressing and cropping the image to accommodate limited storage capacity on the medical device, adding information regarding the attributes of the leads, adding information regarding the image compression and resizing to enable reconstructing the image, and adding information regarding the therapy received and the location of the clinic, for example, where the previous treatment was received, among other information. The user may also use an annotation tool (FIG. 6H) to add annotations to the graphical representation of the lead or leads and the image. For example, the user may add information regarding the configuration or placement of certain leads or the stimulations parameters associated with electrodes on the leads, etc. In some cases, an image may include annotations with respect to a lead revision history, i.e., a history of lead modifications or replacements.

The user may manipulate the leads on the imported image and may manipulate the imported images themselves using the user interface (720). The representations of the leads may be manipulated by the user to match the regions in which they are grouped. Manipulations of the leads representations may be such functions as scaling, stretching, moving, bending, and rotating, for example. Typically, the user may select a lead, e.g., from a menu based on lead type and configuration. The programmer then may add the selected lead to the image displayed by the user interface. The user may reposition the lead, e.g., by dragging the lead or a portion of the lead with a pointing tool such as a stylus, mouse, or the like. In some cases, the user may drag the graphical lead representation, i.e., the drawn lead, to a position that approximately corresponds to a position of an actually-implanted corresponding lead in the image. The lead representation may initially be straight. However, the actual implanted lead or leads shown in the image may be curved, bent or have some other non-linear shape. The representation of the leads on the user interface 59 of the programmer 40 may be also manipulated into a curved, bent, or other nonlinear shape to match the imported image of the region in which the lead is to deliver stimulation therapy. For example, the lead may have control points that the user can click and drag to change the lead from a straight shape to a curved shape. For example, a lead may have multiple control points such that the user may manipulate the lead to have multiple curves. In one example, a lead may have control points on all or a subset of the electrodes associated with the lead. The user may also manipulate the imported images by moving or rotating the image or zooming, for example. The user may manipulate both the drawn graphical representations of the leads and the imported image until the desired placement is achieved, e.g., a placement in which the graphical lead representations are generally aligned with the corresponding imaged leads. In one example, the drawn leads may be represented with a transparent or semi-transparent drawing, so that the user can more easily and accurately align the drawn leads onto the imported image. The orientation of leads in an image may also be automatically detected, and the representation of the drawn leads may be automatically modified and moved to match the leads in the image, saving effort by the user. In one example, the match may be verified visually by the user. In another example, the match may be performed automatically by image processing techniques that identify the leads in the image based on their contrast relative to the background. One or more examples of characterizing leads using image analysis may be described in United States Patent Application No. 12/359,261, published as US 2009-0198306, titled "AUTOMATED PROGRAMMING OF ELECTRICAL STIMULATION ELECTRODES USING POST-IMPLANT IMAGING", filed on January 23, 2009, and United States Application No. 12/359,264, published as US 2009-0196472, titled "ELECTRODE-TO-LEAD ASSOCIATION USING POST-IMPLANT IMAGING", filed on January 23, 2009.

In one example, the user interface may display the image and graphical representation of leads in 2D. In this example, the user interface may display the same region and the corresponding graphical representation of leads implanted in the region in 2D from different perspectives corresponding to different angles or cross sections, as the implanted leads may curve in more than one direction spatially, and allow the user to manipulate the graphical representation of leads for each perspective. In this example, the user may manipulate the leads in the different 2D images corresponding to different perspectives of the same lead arrangement, and the programmer may reconstruct a 3D using the different 2D manipulated images. In another example, the user interface may display the image and graphical representation of leads in 3D. In this example, the user interface may display the region and the corresponding graphical representation of leads implanted in the region in 3D, and may allow the user to rotate and manipulate the image and the graphical representation in all directions.

After a match is completed between the drawn lead layer and the imported image of the corresponding region, including the image leads, the combined image may be compressed and stored for future use (725). The combined image may be imported into and stored on the implanted device for future use by a clinician. A copy of the combined image may be also stored on the patient programmer, a memory device associated with the clinic where patient is receiving therapy, or a network drive, for example. The combined image may then be available for other functions by the programmer (FIG. 6I) such as, for example, delivering therapy to the region through the leads placed in that region.

The process of drawing and matching the leads onto the imported image, then compressing and storing the image may be repeated for each region defined by the user. Compression of the image may be accomplished by using such techniques as cropping, gray-scaling, and reducing intensity/contrast, for example, in different combinations to provide maximum compression.

Subsequently, the creation of therapy programs may occur (per lead region) on the final lead representation of the leads with the image as background, "not shown," or as a sidebar. In other words, the image that was used to position the drawn leads can be displayed with the drawn leads or hidden, or represented in a sidebar region to the side (or above or below) of the graphical image showing the drawn leads. In another example, the user may elect to "hide the leads" and it would appear as though the user is programming directly on the background image. For creation of a therapy program, the user may select electrodes on the graphical drawn leads and assign parameters such as polarity, amplitude, pulse width, and pulse rate. Alternatively, the user may draw stimulation zones within the region, over the leads, and assign stimulation polarities (cathodic or anodic) and stimulation intensities to the leads. In one case, the programmer may automatically compute pertinent stimulation amplitudes, such as pulse current levels, for the electrodes associated with a stimulation zone. The electrodes associated with a stimulation zone may be electrodes that fall partially or entirely within a stimulation zone drawn by the user. The stimulation zone may be a polygon or some other regular or irregular shape. The pulse current levels assigned to electrodes in such a zone may be determined based on relative contributions of the electrodes, according to their positions within the stimulation zone. In another example, the leads may be programmed by selecting leads or parts of leads and defining programming parameters for the selected leads or parts of leads. The parameters may be, for example, amplitude, pulse rate, pulse width, etc.

As shown in the figures, when multiple regions are created, the same leads may be used for all regions, and therefore, one lead may be programmed to provide therapy for more than one region. For example, a lead may be placed near the spine, but may be used to stimulate two regions. For example, one lead may be placed in the epidural spine and another lead may be placed subcutaneously near the epidural lead, one lead region may include both leads together, with a second region including only the epidural lead, and a third lead region utilizing only the subcutaneous lead. The leads may be also divided into groups that are implanted and peripheral, and a combination of implanted and peripheral leads may be used to deliver therapy to one region. In one example, the case electrodes may be also programmed to provide therapy to certain regions, alone or along with other leads (implanted and/or subcutaneous). Each lead may also be programmed so that different criteria, e.g., amplitude, impedance, etc., may be applied to different regions or to different lead groups within a region. The programmer may be capable of providing measurements of inter-region and intra-region signals, to determine the effects of cross signals on the performance of a lead group in a specific region. In one example, the programmer may instruct the IMD to perform measurements, e.g., voltage and current measurements, which the programmer may then use to make determinations regarding the performance of a lead group and characteristics of cross signals between leads. In one example, the programmer may compute an impedance measurement associated with a lead group to determine the group's performance. In this example, the programmer may determine impedance between electrodes using the measurements by the IMD, or information regarding distances between electrodes and body tissue conductivity. In another example, the programmer may determine the inter-region and intra-region measurements based on lead distances, which the programmer may estimate using the images and lead dimension which may be known. In this example, the programmer may disallow a certain combination of electrodes if the lead distance exceeds a specified threshold (e.g., 10 cm). The threshold may be a default value or may be defined by the user. In another example, the programmer may instruct the IMD to measure signal amplitude at one electrode resulting from a stimulation pulse on a second electrode, to determine the effect of the electrodes on one another. In this manner, the IMD may provide measurements to the programmer, which uses the measurements to guide the user as to which electrodes may be programmed together, which electrodes have cross signals, which electrodes may not be programmed together, and the like.

The measurements of inter-region and intra-region signals may be included in the programming of each lead group. For example, if a lead group is programmed to deliver stimulations at certain amplitude, any signal effects resulting from other leads or other regions may be taken into account in presenting the actual stimulation that will be delivered. For example, if there are contributions from other lead groups, a delivered stimulation for a lead group may not be at a specified amplitude, but the overall result of the stimulation actually delivered by the lead may have the desired amplitude. Instead of specifying measurements for each lead, the programmer may allow the user to define an area to which stimulation is to be delivered and to exclude those areas not included in the defined area, and the programmer may configure the leads according to the defined area taking into consideration intra- and inter-region interferences. Additionally, it should be understood that using 4 leads per region and only 4 regions as shown in the figures is only illustrative, and more leads and/or more regions may be defined using the techniques described in this disclosure.

In accordance with this disclosure, some parameters may be global parameters and may be batch programmed for all regions. Global parameters may be, for example, a master rate (the maximum rate at which the programs may run, for example, the master rate may be 100 Hz, and the rate for each program specified per region may defined as a fraction of the master rate), soft start value (the amount of time it takes stimulation to go from 0 to its full amplitude), PW limit on/off (whether the patient can increase or decrease the pulse width - if PW limit is ON, patient is able to increase or decrease the PW, and if it's OFF, then the patient has no control), rate limit on/off (whether the rate limit may be increased or decreased), preferred upper amplitude limit (the upper limit of the amplitude above which a patient may not increase the amplitude, this limit has to be lower or equal to the amplitude (defined next), if the amplitude is 15 mA for example, the preferred upper amplitude limit has to be 15 mA or less), amplitude (the amplitude of the pulse, for example, 25 mA), stimulation on/off (whether the stimulation is on or off), and return to initial settings (this setting may be used if a user adjusts a patient's program during a clinic visit, then for some reason wants to revert to the device settings when the patient came in).

Other parameters may be programmed on region-by-region basis on a region level. Some region level parameters may be, for example, master amplitude (the amplitude for the program specific to the region, and it cannot exceed the preferred upper amplitude limit), electrode amplitude (each electrode of each lead delivering therapy to the region may have its own amplitude), pulse width (the pulse width for the signal of the specific region), slot rate (the fraction of the master rate that defines the rate for the program, this may be a fraction such as, ¼, which means the rate for the specific program is ¼ of the master rate, e.g., 100 Hz, therefore 25 Hz), posture 1/2 amplitude (each slot or program can have its own amplitude, additionally, each posture (standing, sitting, lying down on back, etc.) may have an associated amplitude, when the device senses that the patient changed positions or postures, the associated amplitude is utilized), cycling on/off times (this parameter indicates how long stimulation is on and how long it is off for a specific region), cycling on/off state (this determines whether the cycling feature is on or off, if cycling in on, then the cycling on/off times are applied to the stimulations, and if cycling is off, then stimulation is always on), amplitude upper/lower limits (the upper and lower limits of the amplitude for the program of the specific region, if a patient is able to increase or decrease the amplitude, these limits may be imposed), program name (the name specified by the user for each program, e.g., Region 1, or Epidural Thoracic, etc.), PRS on/off (whether posture amplitudes are applied, when PRS is ON, the amplitude is altered when posture is changed, and when PRS is OFF the amplitude is the same regardless of posture), and delete program. Other parameters may be specified on the global or regional level to accommodate the therapy, the device, and the regions, and are not limited to the exemplary parameters described herein for illustrative purposes.

In an example implementation using the techniques of the disclosure, a user programmer 40 may be used to define one or more therapy regions to which stimulation may be applied by implantable stimulator 34. The stimulation may be applied using previously-defined lead placements or by defining lead placements. Multiple regions may be defined by the user, and for each region one or more leads may be used to deliver stimulation therapy. In one example, a lead may be used to provide stimulation therapy to one or more regions.

The user may graphically define one or more desired stimulation regions using the user interface 59. The user interface may allow a user to view a combined image of the region where the leads may be implanted and graphical layer representing placement of the leads. The image of the region may be retrieved from the implantable stimulator 34 where it may have been stored during a previous session or may be captured by an image capturing device during the current session.

An image capturing device may be used to capture an image of the electrode placement for each of the therapy regions. The image capturing device may be a camera built into the programmer 40 and may be controlled by the user interface 59 or may have its own control panel. Alternatively, the image capturing device may be a camera connected to the programmer 40 via an interface, such as a universal serial bus (USB) interface, or a network interface (e.g., Ethernet, Wi-Fi, or the like).

A captured image may be manipulated by functions such as, for example, zooming, rotating, panning, cropping, and placing annotations on the image. The image may be compressed and other functions such as, cropping and converting to gray scale, for example, may be used to further reduce the size of the image. Metadata may be also associated with the image to enable a subsequent user to retrieve information regarding the region, the applied therapy, and other information related to the patient and the therapy received. The image may then be used to define therapy for a current session, or may be stored in the stimulator 34 for subsequent retrieval for future therapy.

The user may obtain an image by, for example, making a selection on the user interface 59 to capture a screen shot of the image as it appears on the user interface 59. The user may also capture the image using the image capturing device by obtaining a digital photograph off of the screen or a print out of the screen of an imaging machine, e.g., a fluoroscopy machine, which may be connected to the programmer 40. The captured image may be an image, produced by a fluoroscopic imaging device, for example, and may be a still or a moving image.

The captured image may then be manipulated by the user for therapy application. The user may define multiple regions and the lead placement in the captured image. For each region, the user may define a set of leads to use for application of therapy to the region. The user may scale, stretch, move, or rotate the lead images to match the lead placement in the image of the therapy. Additionally, the user may perform other functions such as, for example, zooming, panning, and moving within the image, and adding annotations.

In one example, the therapy may be defined by specifying an electrode combination and specifying parameters associated with the leads and/or electrodes. In another example, the therapy may be defined using zone-based programming, through which the user may graphically define desired stimulation fields and may also define desired therapy intensity. Based on the defined stimulation field and therapy intensity, the contribution of each electrode used in the region may be automatically determined.

Additionally, while aspects of this disclosure are described in the context of a stimulator as an IMD, techniques described herein may be utilized in other types of medical devices which may be implantable. For example, techniques of the disclosure may be utilized with any type of a neurostimulator, or implantable fluid pumps where an image may show catheter configuration, and where therapy is delivered by pumping fluid such as blood, insulin, pain relief agents, or other medicine to the targeted therapy region.

The techniques described in this disclosure may be implemented, at least in part, in hardware, software, firmware or any combination thereof. For example, various aspects of the techniques may be implemented within one or more microprocessors, digital signal processors (DSPs), application specific integrated circuits (ASICs), field programmable gate arrays (FPGAs), or any other equivalent integrated or discrete logic circuitry, as well as any combinations of such components, embodied in programmers, such as physician or patient programmers, stimulators, or other devices. The terms "processor," "processing circuitry," "controller" or "control module" may generally refer to any of the foregoing logic circuitry, alone or in combination with other logic circuitry, or any other equivalent circuitry, and alone or in combination with other digital or analog circuitry.

For aspects implemented in software, at least some of the functionality ascribed to the systems and devices described in this disclosure may be embodied as instructions on a computer-readable medium such as random access memory (RAM), read-only memory (ROM), non-volatile random access memory (NVRAM), electrically erasable programmable read-only memory (EEPROM), FLASH memory, magnetic media, optical media, or the like. The instructions may be executed to support one or more aspects of the functionality described in this disclosure.

Various examples of the invention have been described. These and other examples are within the scope of the following claims.

## Claims

1. A device comprising:
a user interface (59) configured to receive user input; and
a processor (53) configured to associate, in response to user input via the user interface, for each anatomical implant region of a plurality of anatomical implant regions of a patient, one or more implantable elements of an implantable medical device with a respective one of the plurality of anatomical implant regions of the patient; wherein, for each anatomical implant region of the plurality of anatomical regions, the one or more implantable elements associated with the respective one of the plurality of anatomical regions is implanted within the respective one of the plurality of anatomical implant regions, said processor configured to provide, via said user interface, a graphical representation of said one or more implantable elements and the associated anatomical implant regions;
and said user interface (59) further configured to receive user input selecting one of the anatomical implant regions, receive user input specifying one or more therapy parameters for the one or more implantable elements associated with the selected anatomical implant region, and receive user input manipulating a representation of the one or more implantable elements associated with the selected anatomical implant region, wherein the processor is configured to, based on the received user input, at least one of move the representation of the one or more implantable elements associated with the selected anatomical implant region from a first position to a second position or one or more of bend, resize, or shape the representation of the one or more implantable elements associated with the selected anatomical implant region.

2. The device of claim 1, wherein the user interface (59) is configured to present an image of the selected anatomical implant region with the one or more implantable elements associated with the selected anatomical implant region.

3. The device of claim 2, wherein the image comprises a fluoroscopic image.

4. The device of claim 2, further comprising means for obtaining the image in response to a user input.

5. The device of claim 3, wherein the image comprises an image stored in the implantable medical device, the device further comprising means for retrieving the image from the implantable medical device in response to the user input.

6. The device of claim 1, wherein the implantable medical device includes an electrical stimulator (34), the one or more implantable elements include one or more leads (32) that deliver electrical stimulation from the electrical stimulator to the plurality of anatomical implant regions, and the one or more therapy parameters specify one or more therapy parameters for delivery of electrical stimulation by the electrical stimulator via the one or more leads.

7. The device of claim 1, wherein the implantable medical device includes a fluid delivery device, the one or more implantable elements include one or more fluid delivery catheters that deliver the fluid from the device to the one or more anatomical implant regions, and the one or more therapy parameters specify one or more therapy parameters for delivery of fluid by the fluid delivery device via the one or more catheters.

8. The device of claim 1, wherein the plurality of anatomical implant regions include at least a first anatomical implant region and a second anatomical implant region, wherein the user interface is configured to receive user input selecting one of the first and second anatomical implant regions, present a representation of the selected one of the first and second anatomical implant regions with the one or more implantable elements associated with the selected one of the first and second anatomical implant regions, and present user input media to receive the user input specifying the one or more therapy parameters for the one or more implantable elements associated with the selected one of the first and second anatomical implant regions.

9. A computer-readable medium comprising instructions that, upon execution in a device of any preceding claim, cause the processor to:
associate, for each anatomical implant region of a plurality of anatomical regions of a patient, one or more implantable elements of an implantable medical device with a respective one of the plurality of anatomical implant regions of the patient, wherein, for each anatomical implant region of the plurality of anatomical implant regions, the one or more implantable elements associated with the respective one of the plurality of anatomical implant regions is implanted within the respective one of the plurality of anatomical implant regions; and
receive, via a user interface, user input selecting one of the anatomical implant regions;
receive, via the user interface, user input specifying one or more therapy parameter values for the one or more implantable elements associated with the selected anatomical implant region;
receive, via the user interface user input manipulating a representation of the one or more implantable elements associated with the selected anatomical implant region; and
based on the received user input, at least one of move the representation of the one or more implantable elements associated with the selected anatomical implant region from a first position to a second position or one or more of bend, resize, or shape the representation of the one or more implantable elements associated with the selected anatomical implant region.

10. The computer-readable medium of claim 9, further comprising instructions that cause the processor to present an image of the selected anatomical implant region with the one or more implantable elements associated with the selected anatomical implant region.

11. The computer-readable medium of claim 10, wherein the image comprises a fluoroscopic image.

12. The computer-readable medium of claim 10, further comprising instructions that cause the processor to obtain the image in response to a user input.

13. The computer-readable medium of claim 10, wherein the image comprises an image stored in the implantable medical device, the computer-readable medium further comprising instructions that cause the processor to retrieve the image from the implantable medical device.

14. The device of claim 1, wherein the implantable medical device includes an electrical stimulator (34), and the one or more implantable elements include one or more leads (32) that deliver electrical stimulation from the electrical stimulator to the plurality of anatomical implant regions.

15. The computer-readable medium of claim 9, wherein the implantable medical device includes an electrical stimulator (34), and the one or more implantable elements include one or more leads (32) that deliver electrical stimulation from the electrical stimulator to the plurality of anatomical implant regions.

## Patentansprüche

1. Vorrichtung, die enthält:
eine Anwenderschnittstelle (59), die konfiguriert ist, eine Anwendereingabe zu empfangen; und
einen Prozessor (53), der konfiguriert ist, als Reaktion auf eine Anwendereingabe über die Anwenderschnittstelle für jeden anatomischen Implantatbereich von mehreren anatomischen Implantatbereichen eines Patienten ein oder mehrere implantierbare Elemente einer implantierbaren medizinischen Vorrichtung einem entsprechenden der mehreren anatomischen Implantatbereiche des Patienten zuzuordnen;
wobei für jeden anatomischen Implantatbereich der mehreren anatomischen Bereiche das eine oder die mehreren implantierbaren Elemente, die dem entsprechenden der mehreren anatomischen Bereiche zugeordnet sind, innerhalb des entsprechenden der mehreren anatomischen Implantatbereiche implantiert sind, wobei der Prozessor konfiguriert ist, über die Anwenderschnittstelle eine graphische Darstellung des einen oder der mehreren implantierbaren Elemente und der zugeordneten anatomischen Implantatbereiche zu liefern;
und wobei die Anwenderschnittstelle (59) ferner konfiguriert ist, eine Anwendereingabe zu empfangen, die einen der anatomischen Implantatbereiche auswählt, eine Anwendereingabe zu empfangen, die einen oder mehrere Therapieparameter für das eine oder die mehreren implantierbaren Elemente, die dem ausgewählten anatomischen Implantatbereich zugeordnet sind, bestimmt, und eine Anwendereingabe zu empfangen, die eine Darstellung des einen oder der mehreren implantierbaren Elemente, die dem ausgewählten anatomischen Implantatbereich zugeordnet sind, beeinflusst, wobei der Prozessor konfiguriert ist, aufgrund der empfangenen Anwendereingabe die Darstellung des einen oder der mehreren implantierbaren Elemente, die dem ausgewählten anatomischen Implantatbereich zugeordnet sind, von einer ersten Position zu einer zweiten Position zu bewegen und/oder die Darstellung des einen oder der mehreren implantierbaren Elemente, die dem ausgewählten anatomischen Implantatbereich zugeordnet sind, zu biegen und/oder neu zu dimensionieren und/oder zu formen.

2. Vorrichtung nach Anspruch 1, wobei die Anwenderschnittstelle (59) konfiguriert ist, ein Bild des ausgewählten anatomischen Implantatbereichs mit dem einen oder den mehreren implantierbaren Elementen, die dem ausgewählten anatomischen Implantatbereich zugeordnet sind, darzustellen.

3. Vorrichtung nach Anspruch 2, wobei das Bild ein Röntgenbild enthält.

4. Vorrichtung nach Anspruch 2, die ferner Mittel enthält, um das Bild als Reaktion auf eine Anwendereingabe zu erhalten.

5. Vorrichtung nach Anspruch 3, wobei das Bild ein Bild enthält, das in der implantierbaren medizinischen Vorrichtung gespeichert ist, wobei die Vorrichtung ferner Mittel enthält, um das Bild von der implantierbaren medizinischen Vorrichtung als Reaktion auf die Anwendereingabe zu erfassen.

6. Vorrichtung nach Anspruch 1, wobei die implantierbare medizinische Vorrichtung einen elektrischen Stimulator (34) enthält, wobei das eine oder die mehreren implantierbaren Elemente eine oder mehrere Leitungen (32) enthalten, die eine elektrische Stimulation von dem elektrischen Stimulator an die mehreren anatomischen Implantatbereiche verabreichen, und der eine oder die mehreren Therapieparameter einen oder mehrere Therapieparameter für das Verabreichen einer elektrischen Stimulation durch den elektrischen Stimulator über die eine oder die mehreren Leitungen bestimmen.

7. Vorrichtung nach Anspruch 1, wobei die implantierbare medizinische Vorrichtung eine Fluidverabreichungsvorrichtung enthält, wobei das eine oder die mehreren implantierbaren Elemente ein oder mehrere Fluidverabreichungskatheter enthalten, die das Fluid von der Vorrichtung an den einen oder die mehreren anatomischen Implantatbereiche verabreichen, und der eine oder die mehreren Therapieparameter einen oder mehrere Therapieparameter für das Verabreichen von Fluid durch die Fluidverabreichungsvorrichtung über den einen oder die mehreren Katheter bestimmen.

8. Vorrichtung nach Anspruch 1, wobei die mehreren anatomischen Implantatbereiche mindestens einen ersten anatomischen Implantatbereich und einen zweiten anatomischen Implantatbereich enthalten, wobei die Anwenderschnittstelle konfiguriert ist, eine Anwendereingabe zu empfangen, die den ersten oder den zweiten anatomischen Implantatbereich auswählt, eine Darstellung des ausgewählten des ersten und des zweiten anatomischen Implantatbereichs mit dem einen oder den mehreren implantierbaren Elementen, die dem ausgewählten des ersten und des zweiten anatomischen Implantatbereichs zugeordnet sind, darzustellen und Anwendereingabemedien darzustellen, um die Anwendereingabe zu empfangen, die den einen oder die mehreren Therapieparameter für das eine oder die mehreren implantierbaren Elemente, die dem ausgewählten des ersten und des zweiten anatomischen Implantatbereichs zugeordnet sind, bestimmt.

9. Computerlesbares Medium, das Anweisungen enthält, die bei Ausführen in einer Vorrichtung nach einem vorhergehenden Anspruch bewirken, dass der Prozessor:
für jeden anatomischen Implantatbereich von mehreren anatomischen Bereichen eines Patienten ein oder mehrere implantierbare Elemente einer implantierbaren medizinischen Vorrichtung einem entsprechenden der mehreren anatomischen Implantatbereiche des Patienten zuordnet, wobei für jeden anatomischen Implantatbereich der mehreren anatomischen Implantatbereiche das eine oder die mehreren implantierbaren Elemente, die dem entsprechenden der mehreren anatomischen Implantatbereiche zugeordnet sind, innerhalb des entsprechenden der mehreren anatomischen Implantatbereiche implantiert sind; und
über eine Anwenderschnittstelle eine Anwendereingabe zu empfangen, die einen der anatomischen Implantatbereiche auswählt;
über die Anwenderschnittstelle eine Anwendereingabe zu empfangen, die einen oder mehrere Therapieparameterwerte für das eine oder die mehreren implantierbaren Elemente, die dem ausgewählten anatomischen Implantatbereich zugeordnet sind, bestimmt;
über die Anwenderschnittstelle eine Anwendereingabe zu empfangen, die eine Darstellung des einen oder der mehreren implantierbaren Elemente, die dem ausgewählten anatomischen Implantatbereich zugeordnet sind, beeinflusst; und
aufgrund der empfangenen Anwendereingabe die Darstellung des einen oder der mehreren implantierbaren Elemente, die dem ausgewählten anatomischen Implantatbereich zugeordnet sind, von einer ersten Position zu einer zweiten Position bewegt und/oder die Darstellung des einen oder der mehreren implantierbaren Elemente, die dem ausgewählten anatomischen Implantatbereich entsprechen, biegt und/oder neu dimensioniert und/oder formt.

10. Computerlesbares Medium nach Anspruch 9, das ferner Anweisungen enthält, die bewirken, dass der Prozessor ein Bild des ausgewählten anatomischen Implantatbereichs mit dem einen oder den mehreren implantierbaren Elementen, die dem ausgewählten anatomischen Implantatbereich zugeordnet sind, darstellt.

11. Computerlesbares Medium nach Anspruch 10, wobei das Bild ein Röntgenbild enthält.

12. Computerlesbares Medium nach Anspruch 10, das ferner Anweisungen enthält, die bewirken, dass der Prozessor das Bild als Reaktion auf eine Anwendereingabe erfasst.

13. Computerlesbares Medium nach Anspruch 10, wobei das Bild ein Bild enthält, das in der implantierbaren medizinischen Vorrichtung gespeichert ist, wobei das computerlesbare Medium ferner Anweisungen enthält, die bewirken, dass der Prozessor das Bild von der implantierbaren medizinischen Vorrichtung erfasst.

14. Vorrichtung nach Anspruch 1, wobei die implantierbare medizinische Vorrichtung einen elektrischen Stimulator (34) enthält und das eine oder die mehreren implantierbaren Elemente eine oder mehrere Leitungen (32) enthalten, die eine elektrische Stimulation von dem elektrischen Stimulator an die mehreren anatomischen Implantatbereiche verabreichen.

15. Computerlesbares Medium nach Anspruch 9, wobei die implantierbare medizinische Vorrichtung einen elektrischen Stimulator (34) enthält und das eine oder die mehreren implantierbaren Elemente eine oder mehrere Leitungen (32) enthalten, die eine elektrische Stimulation von dem elektrischen Stimulator an die mehreren anatomischen Implantatbereiche verabreichen.

## Revendications

1. Dispositif comportant :
une interface utilisateur (59) configurée pour recevoir une entrée utilisateur ; et
un processeur (53) configuré pour associer, en réponse à une entrée utilisateur via l'interface utilisateur, pour chaque région d'implant anatomique d'une pluralité de régions d'implant anatomique d'un patient, un ou plusieurs éléments implantables d'un dispositif médical implantable à une région respective parmi la pluralité de régions d'implant anatomique du patient ;
dans lequel, pour chaque région d'implant anatomique de la pluralité de régions anatomiques, l'élément ou les éléments implantables associés à la région respective parmi la pluralité de régions anatomiques sont implantés à l'intérieur de la région respective parmi la pluralité de régions d'implant anatomique, ledit processeur étant configuré pour fournir, via ladite interface utilisateur, une représentation graphique dudit ou desdits éléments implantables et des régions d'implant anatomique associées ;
et ladite interface utilisateur (59) étant en outre configurée pour recevoir une entrée utilisateur sélectionnant l'une des régions d'implant anatomique, recevoir une entrée utilisateur spécifiant un ou plusieurs paramètres de thérapie pour l'élément ou les éléments implantables associés à la région d'implant anatomique sélectionnée, et recevoir une entrée utilisateur manipulant une représentation de l'élément ou des éléments implantables associés à la région d'implant anatomique sélectionnée, dans lequel le processeur est configuré pour, sur la base de l'entrée utilisateur reçue, au moins une action parmi déplacer la représentation de l'élément ou des éléments implantables associés à la région d'implant anatomique sélectionnée d'une première position à une seconde position, ou une ou plusieurs actions parmi plier, redimensionner ou former la représentation de l'élément ou des éléments implantables associés à la région d'implant anatomique sélectionnée.

2. Dispositif selon la revendication 1, dans lequel l'interface utilisateur (59) est configurée pour présenter une image de la région d'implant anatomique sélectionnée avec l'élément ou les éléments implantables associés à la région d'implant anatomique sélectionnée.

3. Dispositif selon la revendication 2, dans lequel l'image comporte une image fluoroscopique.

4. Dispositif selon la revendication 2, comportant en outre des moyens pour obtenir l'image en réponse à une entrée utilisateur.

5. Dispositif selon la revendication 3, dans lequel l'image comporte une image mémorisée dans le dispositif médical implantable, le dispositif comportant en outre des moyens pour récupérer l'image à partir du dispositif médical implantable en réponse à l'entrée utilisateur.

6. Dispositif selon la revendication 1, dans lequel le dispositif médical implantable inclut un stimulateur électrique (34), l'élément ou les éléments implantables incluent un ou plusieurs fils (32) qui délivrent une stimulation électrique à partir du stimulateur électrique à la pluralité de régions d'implant anatomique, et le ou les paramètres de thérapie spécifient un ou plusieurs paramètres de thérapie pour la délivrance d'une stimulation électrique par le stimulateur électrique via le ou les fils.

7. Dispositif selon la revendication 1, dans lequel le dispositif médical implantable inclut un dispositif d'administration de fluide, l'élément ou les éléments implantables incluent un ou plusieurs cathéters d'administration de fluide qui administrent le fluide à partir du dispositif à la ou les régions d'implant anatomique, et le ou les paramètres de thérapie spécifient un ou plusieurs paramètres de thérapie pour l'administration de fluide par le dispositif d'administration de fluide via le ou les cathéters.

8. Dispositif selon la revendication 1, dans lequel la pluralité de régions d'implant anatomique inclut au moins une première région d'implant anatomique et une seconde région d'implant anatomique, dans lequel l'interface utilisateur est configurée pour recevoir une entrée utilisateur sélectionnant l'une des première et seconde régions d'implant anatomique, présenter une représentation de la région sélectionnée parmi les première et seconde régions d'implant anatomique avec l'élément ou les éléments implantables associés à la région sélectionnée parmi les première et seconde régions d'implant anatomique, et présenter un support d'entrée utilisateur pour recevoir l'entrée utilisateur spécifiant le ou les paramètres de thérapie pour l'élément ou les éléments implantables associés à la région sélectionnée parmi les première et seconde régions d'implant anatomique.

9. Support lisible par ordinateur comportant des instructions qui, lors d'une exécution dans un dispositif selon l'une quelconque des revendications précédentes, amènent le processeur à :
associer, pour chaque région d'implant anatomique d'une pluralité de régions anatomiques d'un patient, un ou plusieurs éléments implantables d'un dispositif médical implantable à une région respective parmi la pluralité de régions d'implant anatomique du patient, dans lequel, pour chaque région d'implant anatomique de la pluralité de régions d'implant anatomique, l'élément ou les éléments implantables associés à la région respective parmi la pluralité de régions d'implant anatomique sont implantés à l'intérieur de la région respective parmi la pluralité de régions d'implant anatomique ; et
recevoir, via une interface utilisateur, une entrée utilisateur sélectionnant l'une des régions d'implant anatomique ;
recevoir, via l'interface utilisateur, une entrée utilisateur spécifiant une ou plusieurs valeurs de paramètre de thérapie pour l'élément ou les éléments implantables associés à la région d'implant anatomique sélectionnée ;
recevoir, via l'interface utilisateur, une entrée utilisateur manipulant une représentation de l'élément ou des éléments implantables associés à la région d'implant anatomique sélectionnée ; et
sur la base de l'entrée utilisateur reçue, au moins une action parmi déplacer la représentation de l'élément ou des éléments implantables associés à la région d'implant anatomique sélectionnée d'une première position à une seconde position, ou une ou plusieurs actions parmi plier, redimensionner ou former la représentation de l'élément ou des éléments implantables associés à la région d'implant anatomique sélectionnée.

10. Support lisible par ordinateur selon la revendication 9, comportant en outre des instructions qui amènent le processeur à présenter une image de la région d'implant anatomique sélectionnée avec l'élément ou les éléments implantables associés à la région d'implant anatomique sélectionnée.

11. Support lisible par ordinateur selon la revendication 10, dans lequel l'image comporte une image fluoroscopique.

12. Support lisible par ordinateur selon la revendication 10, comportant en outre des instructions qui amènent le processeur à obtenir l'image en réponse à une entrée utilisateur.

13. Support lisible par ordinateur selon la revendication 10, dans lequel l'image comporte une image mémorisée dans le dispositif médical implantable, le support lisible par ordinateur comportant en outre des instructions qui amènent le processeur à récupérer l'image du dispositif médical implantable.

14. Dispositif selon la revendication 1, dans lequel le dispositif médical implantable inclut un stimulateur électrique (34), et l'élément ou les éléments implantables incluent un ou plusieurs fils (32) qui délivrent une stimulation électrique à partir du stimulateur électrique à la pluralité de régions d'implant anatomique.

15. Support lisible par ordinateur selon la revendication 9, dans lequel le dispositif médical implantable inclut un stimulateur électrique (34), et l'élément ou les éléments implantables incluent un ou plusieurs fils (32) qui délivrent une stimulation électrique à partir du stimulateur électrique à la pluralité de régions d'implant anatomique.
